# EUROPEAN PATENT SPECIFICATION

(11) **EP 4 017 496 B1**
(45) Date of publication and mention of the grant of the patent: **07.01.2026**
(21) Application number: 20854153.2
(22) Date of filing: 14.07.2020
(51) Int. Cl.: A61K 31/5415, A61P 27/02, A61P 25/02, A61K 9/00, A61K 9/06, A61K 9/16, A61K 9/50, A61K 47/14, A61K 47/36

(54) **FORMULATIONS FOR USE IN THE PREVENTION AND/OR TREATMENT OF PERIPHERAL NEUROPATHY AND ITS ASSOCIATED DISEASES**
FORMULIERUNGEN ZUR VORBEUGUNG UND/ODER BEHANDLUNG VON PERIPHERER NEUROPATHIE UND DEREN ASSOZIIERTEN ERKRANKUNGEN
FORMULATIONS DESTINÉES À ÊTRE UTILISÉE DANS LA PRÉVENTION ET/OU LE TRAITEMENT DE NEUROPATHIE PÉRIPHÉRIQUE ET SES MALADIES ASSOCIÉES

(30) Priority: 20.08.2019 SG 10201907677Q
(43) Date of publication of application: 29.06.2022
(73) Proprietor: Loh, Yin Sze, Singapore 267858 (SG)
(72) Inventor: Loh, Yin Sze, Singapore 267858 (SG)
(74) Representative: Bardehle Pagenberg Partnerschaft mbB Patentanwälte Rechtsanwälte
(86) International application number: PCT/SG2020/050406
(87) International publication number: WO 2021/034267

(56) References cited:
- WO-A1-2019/016686
- KR-A- 20080 016 372
- US-A1- 2016 310 440
- ROJAS JULIO C. ET AL: "Methylene Blue Provides Behavioral and Metabolic Neuroprotection Against Optic Neuropathy", vol. 15, no. 3, 24 February 2009 (2009-02-24), CH, pages 260 - 273, XP055794473, ISSN: 1029-8428, Retrieved from the Internet <URL:http://link.springer.com/article/10.1007/s12640-009-9027-z/fulltext.html> DOI: 10.1007/s12640-009-9027-z
- AL-AQABA MOUHAMED A ET AL: "Corneal nerves in health and disease", PROGRESS IN RETINAL AND EYE RESEARCH, OXFORD, GB, vol. 73, 7 May 2019 (2019-05-07), XP085933368, ISSN: 1350-9462, [retrieved on 20190507], DOI: 10.1016/J.PRETEYERES.2019.05.003
- POPPERS PAUL J ET AL: "The Effect of Methylene Blue on Neural Tissue", ANESTHESIOLOGY, 1 September 1970 (1970-09-01), pages 335 - 340, Retrieved from the Internet <URL:https://journals.lww.com/anesthesiology/citation/1970/09000/the_effect_of_methylene_blue_on_neural_tissue.14.aspx>
- PEERAER ET AL: "Pharmacological evaluation of rat dorsal root ganglion neurons as an in vitro model for diabetic neuropathy", JOURNAL OF PAIN RESEARCH, vol. 4, 1 February 2011 (2011-02-01), GB, pages 55 - 65, XP093298453, ISSN: 1178-7090, DOI: 10.2147/JPR.S15452
- "PRISOLINE BLUE EYE DROPS 10 ML", YAOOTA, 18 August 2014 (2014-08-18), XP055794469, Retrieved from the Internet <URL:https://yaoota.com/en-eg/product/prisoline-blue-eye-drops-10-ml-price-from-seif-egypt> [retrieved on 20201030]
- TATU, ALIN L., ARDELEANU VALERIU, ELISEI ALINA M., DUMITRIU BUZIA OLIMPIA, MIULESCU MAGDALENA, NWABUDIKE LAWRENCE C.: "Undesirable Effects of Some Topical Antiseptics. Chemical, pharmacological and dermatological aspects", REVISTA DE CHIMIE, vol. 70, no. 6, 15 July 2019 (2019-07-15), pages 2276 - 2281, XP055794470, DOI: 10.37358/RC.19.6.7322
- DAUDT DONALD R., MUELLER BRETT, PARK YONG H., WEN YI, YORIO THOMAS: "Methylene Blue Protects Primary Rat Retinal Ganglion Cells from Cellular Senescence", INVESTIGATIVE OPHTHALMOLOGY & VISUAL SCIENCE, vol. 53, no. 8, 31 July 2012 (2012-07-31), pages 4657 - 4667, XP055794471, DOI: 10.1167/iovs.12-9734
- ROJAS, JULIO C., JOHN JOSEPH M., LEE JUNG, GONZALEZ-LIMA F.: "Methylene blue provides behavioral and metabolic neuroprotection against optic neuropathy", NEUROTOXICITY RESEARCH, vol. 15, no. 3, 24 February 2009 (2009-02-24), pages 260 - 273, XP055794473, DOI: 10.1007/S12640-009-9027-Z
- BARANOWSKI, P. ET AL.: "Ophthalmic Drug Dosage Forms: Characterisation and Research Methods", THE SCIENTIFIC WORLD JOURNAL, vol. 2014, 18 March 2014 (2014-03-18), pages 1 - 14, XP055643530, [retrieved on 20201030], DOI: 10.1155/2014/861904

## Description

### . CROSS REFERENCE TO RELATED APPLICATIONS

. This application claims the priority to Singapore patent application No. 10201907677Q, filed 20 August 2019.

### . Field

. The invention relates to formulations and their use in the prevention and/or treatment of peripheral neuropathy and its associated diseases, particularly in the eye.

### . Background

. The following discussion of the background to the invention is intended to facilitate understanding of the present invention.

. Our nervous system is divided in two components: the central nervous system (CNS), which includes the brain and the spinal cord, and the peripheral nervous system (PNS), which encompasses the dorsal root ganglions(DRG) and also nerves fibres outside the brain and spinal cord and the nerve receptors terminally. The PNS consists of 2 divisions, the afferent (sensory) division which sends sensory information towards the CNS, and the efferent (motor) division, which conveys motor commands from the CNS to the effector targets (cells, tissues.etc). The PNS allows the CNS to communicate with the external environment.

. Unlike the brain and the spinal cord of the CNS that are protected by myelin layers, the vertebrae and the skull, the nerves of the PNS are sometimes unmyelinated. Neurons are the cell bodies constituting the nervous system. Each neuron has a long process, known as the axon, which transmits the electrochemical signals through which neurons communicate. These axonal processes constitute the PNS. The Dorsal Root Ganglion is part of the PNS and its nerve fibres conduct information from sensory inputs to go eventually to the brain for processing. Peripheral nerves supply important sensory units in the hands, feet and also the nerves of the cornea. Peripheral neuropathy is damage to the PNS. Peripheral neuropathy, often causes weakness, numbness and pain. Peripheral neuropathy can result from traumatic injuries, infections, autoimmune conditions, metabolic problems, inherited causes, ageing and exposure to toxins. One of the most common causes is diabetes which results in hyperglycemia/ raised glucose level in the human body. Hyperosmolarity is also a sequelae of hyperglycemia potentially leading to hyperosmolarity induced peripheral nerve damage.

. Diabetes Mellitus (DM), is a group of metabolic disorders characterised by high blood sugar levels over prolonged period of time. As of 2017, an estimated 425 million people had diabetes worldwide (Diabetes atlas 2017). In Diabetes Mellitus (DM), there is associated complications of peripheral neuropathy or damage to the PNS. Diabetic peripheral neuropathy is the one of the most common complication in DM and affects all peripheral nerves including sensory neurons and motor neurons and less often affects the autonomic nervous system. Currently, there is no definitive management for hyperglycemia (high glucose) induced peripheral nerve injury besides good control of blood sugar level. Managing blood sugar levels can be very difficult for some patients especially those who have to be on insulin injections, due to cost, and pain of injections. With the exception of managing blood sugar levels the only treatment for diabetic neuropathy is reducing pain and other symptoms. Degradation of nerve fibres is common in type II diabetes, particularly in diabetes patients with proliferative diabetic retinopathy (Gao et al. Int J Ophthalmol, Vol 8, No.2, 2015, 358-364). Patients with DM demonstrate signs such as a progressive decrease in corneal nerve density and reduction in corneal sensitivity (Han et al. Clinical interventions in Aging 2019: 14 p 53-63). Corneal nerve fibre length measured with confocal microscopy has been demonstrated to be capable of predicting the development of diabetic peripheral neuropathy (Han et al 2019). Due to hyperglycemia, the ocular surface environment can also become hyperosmolar, resulting in hyperosmolarity induced corneal nerve damage. There is a need for better management and treatment of diabetic neuropathy such preventing and/or treating peripheral nerve damage in the eye, especially the corneal nerve and its branches.

. Methylthioninium Chloride (MTC) (also known as Methylene blue (MB); methylthionine chloride; tetramethylthionine chloride; 3,7-bis(dimethylamino) phenothiazin-5-ium chloride; C.I. Basic Blue 9; tetramethylthionine chloride; 3,7-bis(dimethylamino) phenazathionium chloride; Swiss blue; C.I. 52015; C.I. Solvent Blue 8; aniline violet; and Urolene Blue^{®}) is a low molecular weight (319.86), water soluble, tricyclic organic compound of the following formula:

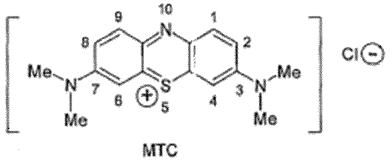

. Various synthesis methods for MTC are known and have been summarized in WO 2006/032879. WO 2006/032879 also discloses a number of applications of methylene blue, which include use as a medical dye, as a redox indicator, as an antiseptic, for the treatment and prevention of kidney stones, and for the treatment of melanoma, malaria, and viral infections. MTC dosed systemically has also been used as an oxidizing/reducing ( redox) agent and as an antidote in the case of carbon monoxide, nitrite and aniline poisoning. Systemic MTC has also been proposed for treatment of Alzheimer's disease, a condition affecting the central nervous system.

Rojas Julio C. et al, Neurotoxicity Research,vol. 15, no. 3, 24 February 2009, discloses intravitreal injection of MB in DMSO to prevent neurotoxicity of rotenone in retinal ganglia.Daubt Donald R. et al, Investigative Ophthalmology & Visual Science, vol. 53, no. 8, 31 July 2012, pages 4657-4667 discloses that MB protects primary rat retinal ganglion cells from cellular senescence. KR20080016372 discloses the use of MB for the treatment of retinal damage.

. MTC is soluble in water and alcohol. MTC is not recommended to be dissolved in saline because precipitation has been reported in cases where methylene blue has been diluted with sodium chloride 0.9%, saline (due to presence of chloride ions which have been shown to reduce the solubility of methylene blue). MTC is reported to be incompatible with caustic alkalis, iodides and dichromates, and oxidising and reducing substances.

. MTC when injected into the spinal cord compartment of cerebral spinal fluid has been reported to cause massive peripheral nerves damage and resultant total paraplegia (Sharr et al. J. Neurol. Neurosur, and Psychiatry 1978, 41, 384-386).

. Accidental use of MTC on the eye in higher concentrations, instead of Trypan Blue, during cataract surgery has led to irreversible blindness, corneal edema (Timucin et al. Arq. Bras. Oftalmol. vol.79 no.2, 2016), bullous keratopathy resulting in keratoplasty, and endophthalmitis with progression to corneal failure. Methylene blue is therefore generally considered a toxic dye not for use in the eyes. Further, as MTC is a dye its use on skin or mucosa generally results in unsightly blue discoloration and stains.

. Thus, there exists a need to develop formulations methods and uses which ameliorates at least one of the disadvantages outlined above.

### . Summary

. An object of the invention is to ameliorate some of the above mentioned difficulties preferably with formulations and methods of using formulations in the prevention and/or treatment of peripheral neuropathy, particularly in the eye.

The present invention relates to a topical preparation for use in preventing and/or treating peripheral nerve damage on body surfaces including mucous membranes or a nerve site on the surface of the cornea comprising an effective amount of 3,7-bis(dimethylamino)phenothiazin-5-ium chloride and a carrier-medium as defined in the claims.

. One non-claimed aspect of the present disclosure relates to a topical preparation for preventing and/or treating peripheral nerve damage and its associated conditions comprising, an effective amount of diaminophenothiazine compound or tautomeric forms of formula I: 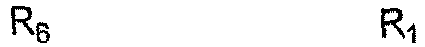 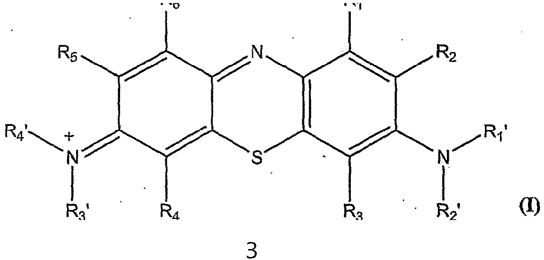 wherein R₁, R₂, R₃, R₄, R₅, R₆, R₁', R₂', R₃', and R₄' are independently hydrogen, methyl or ethyl.

. Another non-claimed aspect of the present disclosure relates to a topical preparation as described herein above, for use in preventing and/or treating peripheral nerve damage and its associated conditions.

. Another non-claimed aspect of the present disclosure relates to a method of preventing and/or treating peripheral nerve damage comprising administering to a subject in need of preventing and/or treating peripheral nerve damage and its associated conditions
(a) an effective amount of diaminophenothiazine compound or tautomeric forms of formula I 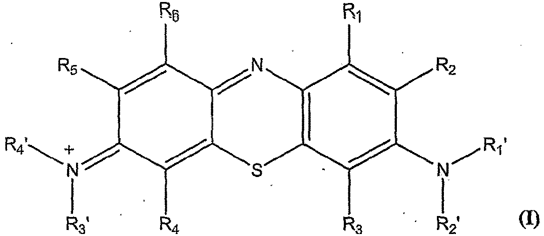 wherein R₁, R₂, R₃, R₄, R₅, R₆, R₁', R₂', R₃', and R₄' are independently hydrogen, methyl or ethyl;
   or
(b) a topical preparation as described herein above, or
(c) a topical preparation comprising, an effective amount of diaminophenothiazine compound or tautomeric forms of formula I; and a carrier.

. Another non-claimed aspect of the present disclosure relates to use of a compound of formula I, in the manufacture of a topical preparation for prevention and/ or treatment of peripheral nerve damage and its associated conditions.

. Another aspect of the invention relates to a topical preparation for preventing and/or treating peripheral nerve damage comprising, less than or equal to 150 µM of 3,7-bis(dimethylamino) phenothiazin-5-ium chloride; and a carrier-medium that adjusts the viscosity of the preparation to a range of about 15 to 150 milli-Pascals per second as defined in the claims.

. Another aspect of the invention relates to a topical kit comprising a) a topical composition comprising of an effective amount of 3,7-bis(dimethylamino) phenothiazin-5-ium chloride , and b) a container for holding the said composition for use in a method of preventing and/or treating peripheral nerve damage on body surfaces including mucous membranes or a nerve site on the surface of the cornea, more specifically on the eye relating to corneal nerve injury and its associated conditions including neurotrophic keratopathy.

. Another non-claimed aspect of the present disclosure relates to a method of formulating a topical composition comprising: encapsulating 3,7-bis(dimethylamino) phenothiazin-5-ium chloride in a microparticle.

. Other aspects and features of the present invention will become apparent to those of ordinary skill in the art upon review of the following description of specific embodiments of the invention in conjunction with the accompanying figures.

### . Brief description of the drawings

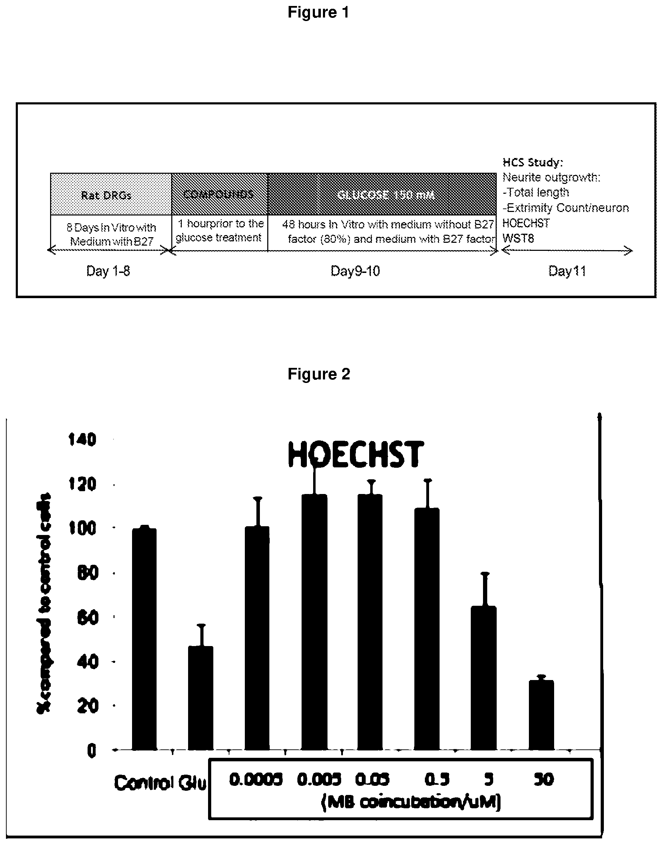

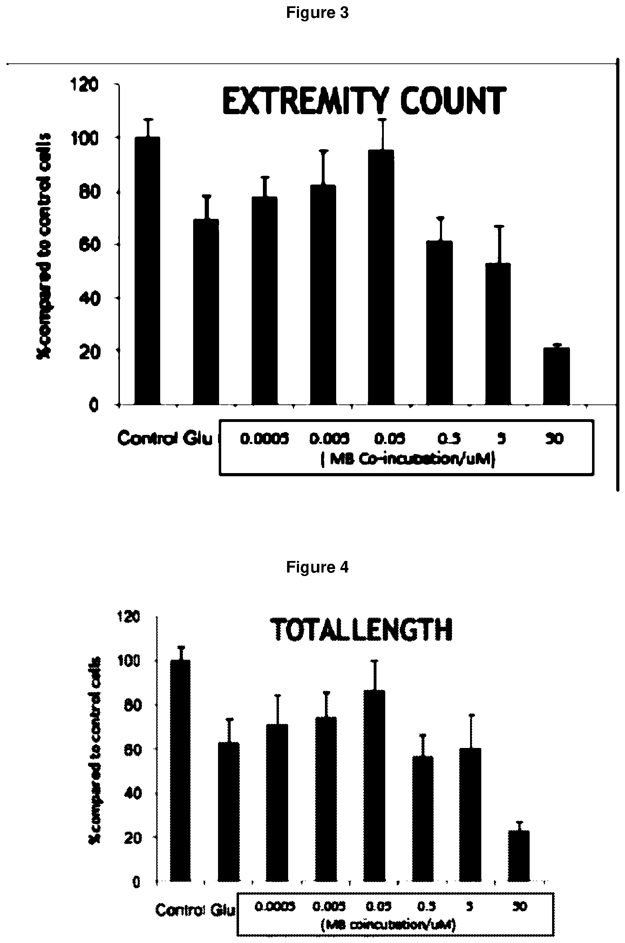

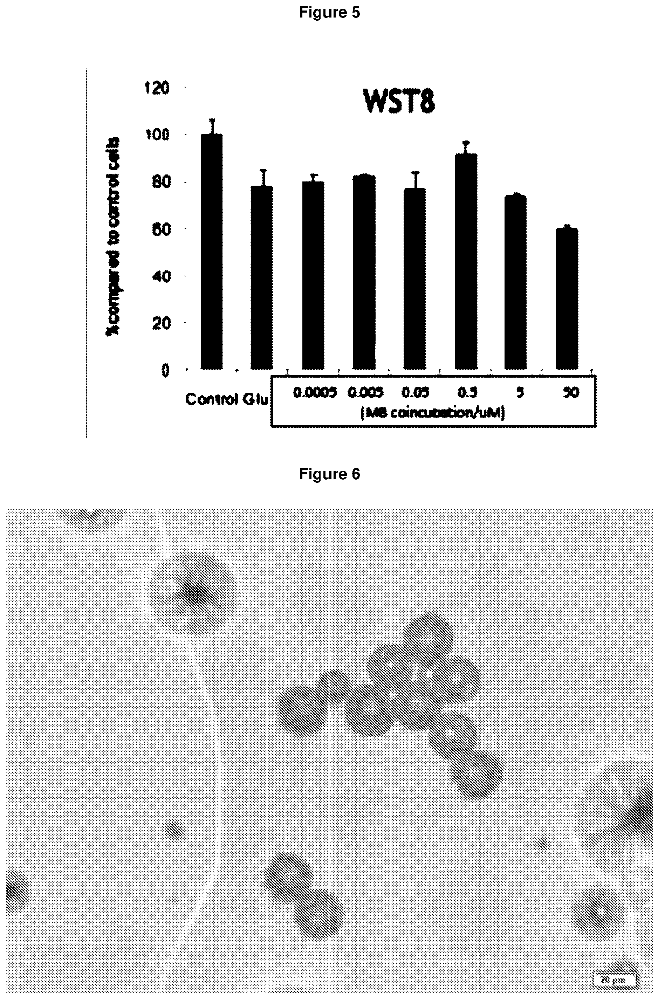

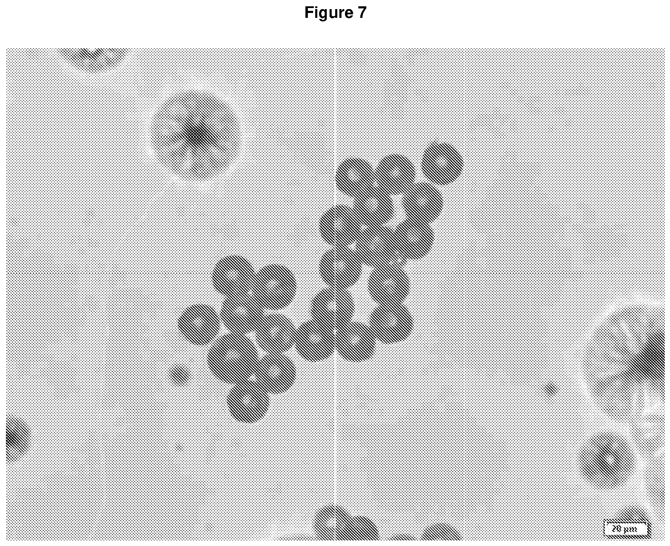

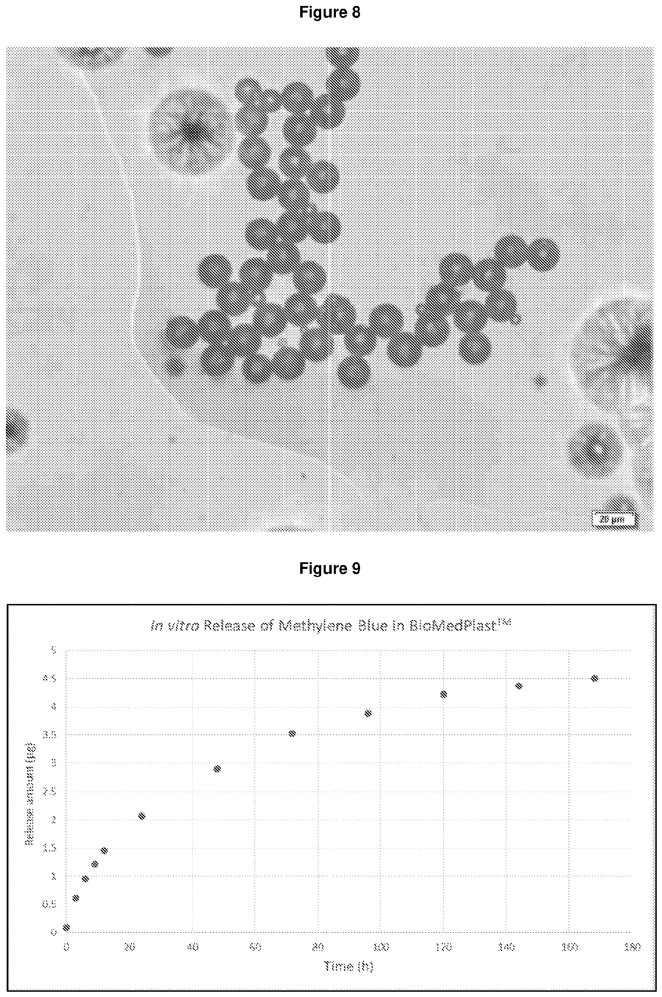

. In the figures, which illustrate, by way of example only, embodiments of the present invention include,
. **Figure 1****.** Schematic of the test timeline.
. **Figure 2****.** Cell viability by hoechst 33342 nucleic acid stain in cells treated with methylene blue under glucose toxicity condition. DRGs were treated with methylene blue 1h prior to 150 mM glucose and 48h later HTS assay was performed.
. **Figure** 3. Extremity number per neuron by beta III tubulin staining in cells treated with methylene blue under glucose toxicity condition. DRGs were treated with methylene blue 1h prior to 150 mM glucose and 48h later HTS assay was performed.
. **Figure 4****.** Total length per neuron by beta III tubulin staining in cells treated with methylene blue under glucose toxicity condition. DRGs were treated with methylene blue 1h prior to 150 mM glucose and 48h later HTS assay was performed.
. **Figure 5****.** Cell viability by WST8 staining in cells treated with methylene blue under glucose toxicity condition. DRGs were treated with methylene blue 1h prior to 150 mM glucose and 48h later HTS assay was performed.
. **Figure 6****:** Sample microscope image of microspheres manufactured in Run 1 (40 × magnification).
. **Figure 7****:** Sample microscope image of microspheres manufactured in Run 2 (40 × magnification).
. **Figure 8****:** Sample microscope image of microspheres manufactured in Run 3 (40 × magnification).
. **Figure 9****:** Graph of the 7-day release profile of methylene blue encapsulated microspheres on 5 mg of sample produced from Run 2.

### . Detailed description

Note that the scope of the invention is defined by the claims. Any subject-matter falling outside the scope of the claims is provided for information purposes only. Any references to the methods of treatment by therapy of this description are to be interpreted as references to compounds, pharmaceutical compositions and medicaments of the present invention for use in those methods.

. Particular embodiments of the present invention will now be described with reference to the accompanying drawings. The terminology used herein is for the purpose of describing particular embodiments only and is not intended to limit the scope of the present invention. Additionally, unless defined otherwise, all technical and scientific terms used herein have the same meanings as commonly understood by one of ordinary skill in the art to which the present invention belongs. Where possible, the same reference numerals are used throughout the figures for clarity and consistency.

. Various embodiments relate to a topical preparation for preventing and/or treating peripheral nerve damage and its associated conditions comprising, a diaminophenothiazine compound or tautomeric forms of formula I: 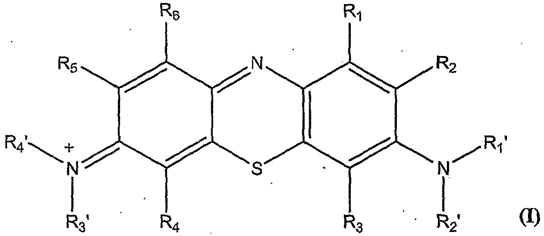 wherein R₁, R₂, R₃, R₄, R₅, R₆, R₁', R₂', R₃', and R₄' are independently hydrogen, methyl or ethyl.

. In various embodiment, the forms of compounds of formula (1), include pharmaceutically acceptable salts, hydrates, and solvates thereof.

. Various embodiments relate to a topical preparation for protecting against preventing and/or treating peripheral nerve damage and its associated conditions comprising, an effective amount of diaminophenothiazine compound or tautomeric forms of formula I:
wherein R1, R2, R3, R4, R5, R6, R1', R2', R3', and R4' are independently hydrogen, methyl or ethyl less than or equal to 5 µM of 3,7-bis(dimethylamino) phenothiazin-5-ium chloride; and a physiological buffer.

. As used herein Diaminophenothiazine compounds are positively charged due to electron delocalization resulting in partial positive charges located on both nitrogen and sulfur atoms. In various embodiments, scheme A shows the chemical structure of thionine, which is the simplest diaminophenothiazine, and the three tautomeric forms of thionine created by electron delocalization:

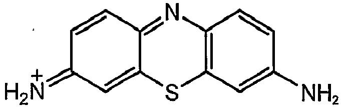

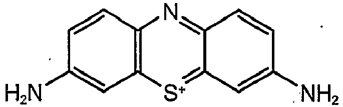

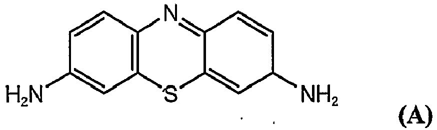

. In various embodiments, R₁, R₂, R₃, R₄, R₅, and R₆ are hydrogen, and R₂, R₅, R₁ ¹, R₂', R₃', and R₄ ¹ are independently hydrogen or methyl.

. In various embodiments, the diaminophenothiazine compound, also sometimes called thiazins, is selected from the group consisting of azure A, azure B, azure C, thionine, toluidine blue, 3,7-bis(dimethylamino) phenothiazin-5-ium chloride (methylene blue), new methylene blue, and 1-9-dimethyl methylene blue.

. According to the present invention, the diaminophenothiazine compound is 3,7-bis(dimethylamino) phenothiazin-5-ium chloride.

. In various embodiments, the diaminophenothiazine compound comprises less than or equal to 150 µM 3,7-bis(dimethylamino) phenothiazin-5-ium chloride.

. In various embodiments, the 3,7-bis(dimethylamino) phenothiazin-5-ium chloride comprises the compound of formula II:

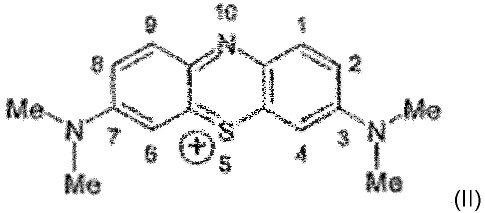

. While, methylene blue in the management of any eye related conditions has been strictly discouraged or prohibited, it has been devised that a solution having a significantly lower concentration of methylene blue that has been buffered to a physiological pH range can be tolerated. Similarly, the solution can be tolerated by peripheral nerves. Advantageously, a lower concentration of methylene blue can be used for management of peripheral nerve diseases, particularly in various embodiments as a topical preparation in an eye related peripheral nerve disease and its associated conditions that may be caused by traumatic injuries, infections, autoimmune conditions, metabolic problems, inherited causes, ageing, exposure to toxins such as chemotherapy, hyperglycemia and/or hyperosmolarity induced peripheral nerve damage, or any other cause known in the art that results in peripheral nerve damage and its associated conditions.

. As used herein the term 'topical preparation' refers to any preparation for application to a particular place in or on a body. It may refer to solutions, suspensions, lotions, ointments, gels, powders, pastes, matrixes, transdermal patches or any other formulation that allows application to a particular place in or on a body. In various embodiments the particular place in or on a body refers to body surfaces such as skin or mucous membranes. In various embodiments the particular place in or on a body refers to directly at a nerve site for example a nerve site on the surface of the cornea. In various embodiments the particular place in or on a body refers to the eye.

. As used herein the term 'prevention', 'preventing', 'prevent', 'protecting', 'protect', or 'protection' of peripheral nerve damage refers to preventing against, blocking or reducing damage or death of nerve cells, fibers or extensions from the PNS. In various embodiments this may be achieved by reducing the negative sequelae resulting from the influx of glucose into the mitochondria of nerve cells resulting in cellular injury. Nerve cells are known to have greater mitochondrial volume than other cells. In various embodiments this may be achieved by reducing non-enzymatic bonding i.e. glycation of glucose to proteins or lipoproteins that may lead to damage of peripheral nerves. In various embodiments this may be achieved by reducing the toxic effects or toxins such as reducing the undesirable effects of chemotherapeutic drugs on nerves. In various embodiments this may be achieved by increasing the anti-oxidant defense capacity of the cell.

. As used herein the term ' 'treating', 'treat' or 'treatment' of peripheral nerve damage refers to stopping or reducing further damage or death of nerve cells, fibers or extensions from the PNS and/or providing an environment suitable for formation and growth of new nerve cells, fibers or extensions in the PNS. In various embodiments this may be achieved by reducing the negative sequelae resulting from the influx of glucose into the mitochondria of nerve cells resulting in cellular injury. Nerve cells are known to have greater mitochondrial volume than other cells. In various embodiments this may be achieved by reducing non-enzymatic bonding i.e. glycation of glucose to proteins or lipoproteins that may lead to damage of peripheral nerves. In various embodiments this may be achieved by reducing the toxic effects or toxins such as reducing the undesirable effects of chemotherapeutic drugs on nerves. In various embodiments this may be achieved by increasing the anti-oxidant defense capacity of the cell and/or its nerve fibres.

. As used herein the term '3,7-bis(dimethylamino) phenothiazin-5-ium chloride' may refer to Methythioninium Chloride (MTC) (also known as Methylene blue (MB); methylthionine chloride; tetramethylthionine chloride; C.I. Basic Blue 9; tetramethylthionine chloride; 3,7-bis(dimethylamino) phenazathionium chloride; Swiss blue; C.I. 52015; C.I. Solvent Blue 8; aniline violet; and Urolene Blue^{®}) comprising a low molecular weight (319.86), water soluble, tricyclic organic compound of the following formula:

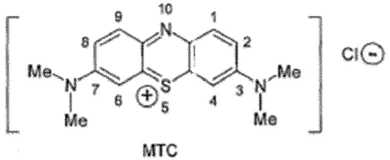

. In various embodiments the 3,7-bis(dimethylamino) phenothiazin-5-ium chloride may be formulated by any of the methods known in the art.

. In various embodiments the topical preparation comprises between 0.0001 µM and 150 µM of 3,7-bis(dimethylamino) phenothiazin-5-ium chloride. In various embodiments the topical preparation comprises 149.5 µM or less of MTC. In various embodiments the topical preparation comprises 59.5µM or less of MTC. In various embodiments the topical preparation comprises 4.5 µM or less of MTC. In various embodiments the topical preparation comprises between 0.0001 µM and 50 µM of 3,7-bis(dimethylamino) phenothiazin-5-ium chloride. In various embodiments the topical preparation comprises between 0.001 µM and 90 µM, or between 0.005 µM and 0.5 µM or between 0.0059 µM and 0.49 µM of 3,7-bis(dimethylamino) phenothiazin-5-ium chloride. In various embodiments the topical preparation is selected from the group comprising 0.0005 µM, 0.005 µM, 0.05 µM 0.5 µM, 5µM, 50µM, 100µM and 150µM of 3,7-bis(dimethylamino) phenothiazin-5-ium chloride. In various embodiments the topical preparation comprises 0.05 µM of 3,7-bis(dimethylamino) phenothiazin-5-ium chloride.

. In various embodiments, the topical preparation further comprising a physiological buffer.

. In various embodiments the physiological buffer comprises a hydrogen ion aqueous solution comprising a mixture of an acid and its conjugate base capable of maintaining a preparation at a physiological pH of about pH 4 to about pH8 when it comes into contact with additives or components of a biological environment. In various embodiments the physiological buffer is selected from the group comprising bicarbonate, bicine. Cacodylate, HEPES, MES, MOPS, PIPES, phosphate, TEPS, TAPSO, TES, Tricine, and Tris. In various embodiments the physiological buffer comprises sodium bicarbonate and sodium chloride. Surprisingly, despite technical prohibition to use MTC with sodium chloride when the sodium chloride is present together with sodium bicarbonate in a physiological buffer the diaminophenothiazine compound such as MTC remains in solution, demonstrates protection of peripheral nerves and has a pH suitable for both direct use on peripheral nerves and ophthalmic use. In various embodiments the pH of the solution is from 4 to 8. In various embodiments the pH of the solution is from 4 to 7.5. In various embodiments the pH of the solution is 4. In various embodiments the pH of the solution is 5. In various embodiments the pH of the solution is 6. In various embodiments the pH of the solution is 7. In various embodiments the pH of the solution is 7.4. In various embodiments the pH of the solution is 7.5. In various embodiments the pH of the solution is 8.

. In various embodiments the physiological buffer solution comprises HEPES (4-(2-hydroxyethyl)-1-piperazineethanesulfonic acid), preferably in the presence of sodium bicarbonate and sodium chloride. In various embodiments the physiological buffer comprises a neurobasal medium.

. The topical preparation comprises a carrier. The carrier is a carrier-medium, which may be liquid and/or aqeous in nature. In various embodiments the carrier or carrier-medium may comprise polymers such as hydrophilic polymers of high molecular weight, polyvinyl alcohol, poloxamers, hyaluronic acid, carbomers, cellulose derivatives (gellan gum, xanthan gum), 2-hydroxypropyl-beta-cyclodextrin, poly(2-hydroxyethylmethacrylate), ethylene glycol dimethylacrylate, acrylamide, N-vinylpryrrolidone and ethylacrylate or combinations thereof. In various embodiments the carrier or carrier-medium may comprise excipients that may increase drug delivery into the eye such as chelating agents, surfactants or cyclodextrins. In various embodiments the carrier or carrier-medium may comprise inclusion complexes such as nanoparticles, microparticles, niosomes, discosomes, dendrimers, in situ gels, wafers or embedded in contact lenses. In various embodiments the carrier or carrier - medium may comprise sugar molecules such as sucrose, glucose, lactose D-glucose (dextrose). In various embodiments the endogenous glucose naturally occurring in the peripheral nerve environment may form part of the carrier or carrier-medium in solution. This is particularly relevant in subjects with DM who have hyperglycemia involving a high concentration of glucose.

. In various embodiments the carrier or carrier medium comprises a microparticle or microspheres.

. In various embodiments, the effective amount of diaminophenothiazine compound comprises 3,7-bis(dimethylamino) phenothiazin-5-ium chloride pre-loaded in the microparticle.

. In various embodiments, the microparticle is coated with a mucoadhesive agent such as chitosan. In various embodiments, the microparticle is suspended in a fluid medium. In various embodiments, the fluid medium is aqueous in nature such that the diaminophenothiazine compound 3,7-bis(dimethylamino) phenothiazin-5-ium chloride is capable of dissolution in the said fluid medium.

. In various embodiments the carrier or carrier medium adjusts viscosity of the topical preparation to a range of about 15 to 150 milli-Pascals per second.

. In various embodiments the carrier or carrier medium modulates the release profile of the diaminophenothiazine compound.

. In various embodiments the carrier or carrier-medium modulates the release profile of the diaminophenothiazine compound such as 3,7-bis(dimethylamino) phenothiazin-5-ium chloride over a pre-determined time period. Any known carrier or carrier-medium that is able to modulate the rate of the diaminophenothiazine compound such as MTC release into the peripheral nerve environment would be suitable. In various embodiments the carrier or carrier-medium modulates the release profile of 3,7-bis(dimethylamino) phenothiazin-5-ium chloride to a predetermined rate below or slower than the rate of release of 3,7-bis(dimethylamino) phenothiazin-5-ium chloride per se. In various embodiments a suitable carrier or carrier-medium may comprise polymers such as hydrophilic polymers of high molecular weight, polyvinyl alcohol, poloxamers, hyaluronic acid, carbomers, cellulose derivatives (gellan gum, xanthan gum), 2-hydroxypropyl-beta-cyclodextrin, poly(2-hydroxyethylmethacrylate), ethylene glycol dimethylacrylate, acrylamide, N-vinylpryrrolidone and ethylacrylate or combinations thereof. In various embodiments a suitable carrier or carrier-medium may comprise inclusion complexes such as nanoparticles, microparticles, niosomes, discosomes, dendrimers, in situ gels, wafers or may be embedded in contact lenses. In various embodiments the carrier or carrier-medium slows down or reduces the release profile of the diaminophenothiazine compound such as 3,7-bis(dimethylamino) phenothiazin-5-ium chloride over a pre-determined time period.

. In various embodiments the carrier or the carrier-medium adjusts viscosity of the topical preparation to a range of about 15 to 150 milli-Pascals per second. In various embodiments the carrier or carrier medium adjusts, increases the viscosity to achieve a range of about 15 to 150 milli-Pascals per second. In various embodiments the carrier or carrier-medium increases the viscosity to a range of about 15 to 100 milli-Pascals per second. In various embodiments the carrier or carrier-medium adjusts, increases the viscosity to achieve a range of about 15 to 50 milli-Pascals per second. In various embodiments the viscosities are those at room temperature. Increased viscosity may have the advantage of enabling longer contact time between the diaminophenothiazine compound such as MTC and the corneal surface, thus increasing its bioavailability especially to the corneal nerve and its branches. In various embodiments the polymers, sugar molecules, nanoparticles, microparticles, niosomes, discosomes, dendrimers carriers or carrier-medium may adjust or increase the viscosity of the solution.

. In various embodiments the carrier or carrier-medium modulates the release profile of the diaminophenothiazine compound such as 3,7-bis(dimethylamino) phenothiazin-5-ium chloride. In various embodiments the carrier or carrier-medium comprises a micro-particle with a sustained release profile. This has the advantage of increasing the viscosity of the preparation and it also reduces the need for frequent applications of eye drops by increasing the pre-corneal residence or retention time of the topical preparation. The sustained release formulation also has the advantage of reducing unsightly blue stains on the skin as the increased viscosity reduces dripping and/or release with tears causing less stains and discoloration on the face. In various embodiments the carrier or carrier-medium micro-particle with a sustained release profile comprises a poly (lactide-co-glycolide) (PLGA) microspheres however any microparticle known in the art to be able to provide a slow/ sustained release profile would be suitable. In various embodiments the PLGA microspheres comprise a polymer ratio of PLGA (50:50), PLGA (75:25), or PLGA (85:15). Such ratios may be able to modulate the release profile of the diaminophenothiazine compound such as MTC. In various embodiments the sustained release profile of the diaminophenothiazine compound such as MTC may be modulated to 1, 3, or 6 months with the use of different polymer ratios in the microspheres, or different sizes of microspheres.

. In various embodiments, the topical preparation, further comprising one or more additional compounds, including drugs.

. Various embodiments relate to a topical preparation as described herein, for use in preventing and/or treating peripheral nerve damage.

. In various embodiments the topical preparation is suitable for use in animals including humans and may be suitable for prevention and/or treatment of peripheral nerve damage in medical or veterinary settings.

. In various embodiments the peripheral nerve damage results in or comprises neurotrophic keratopathy. In various embodiments the peripheral nerve damage comprises peripheral neuropathy caused by traumatic injuries, infections, autoimmune conditions, metabolic problems, inherited causes, ageing, exposure to toxins such as chemotherapy, hyperglycemia induced peripheral nerve damage, or any other cause known in the art that results in peripheral nerve damage.

. In various embodiments the topical preparation is for ophthalmic use. In various embodiments the topical preparation is in the form of eye drops or ointment.

. Various embodiments relate to a method of preventing and/or treating peripheral nerve damage comprising administering to a subject in need of preventing and/or treating peripheral nerve damage
(a) a diaminophenothiazine compound or tautomeric forms of formula I 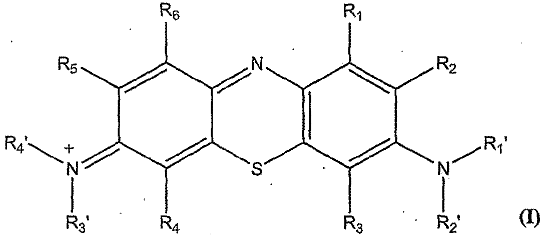 wherein R1, R2, R3, R4, R5, R6, R1', R2', R3', and R4' are independently hydrogen, methyl or ethyl; or
(b) a topical preparation according to any one of claims 1 to 13, or
(c) a topical preparation comprising, a diaminophenothiazine compound or tautomeric forms of formula I; and a carrier or carrier-medium.

. Various embodiments relate to a method of preventing and/or treating peripheral nerve damage and its associated conditions comprising administering to a subject in need of preventing and/or treating peripheral nerve damage and its associated conditions
(a) an effective amount of diaminophenothiazine compound or tautomeric forms of formula I 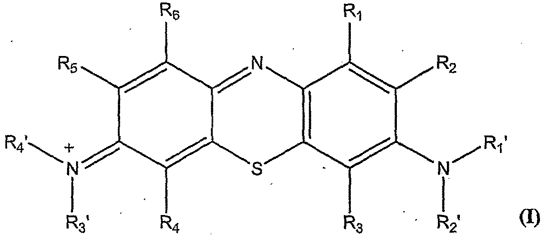 wherein R₁, R₂, R₃, R₄, R₅, R₆, R₁', R₂', R₃', and R₄' are independently hydrogen, methyl or ethyl;
   or
(b) a topical preparation according to any one of claims 1 to 13, or
(c) a topical preparation comprising, an effective amount of diaminophenothiazine compound or tautomeric forms of formula I; and a carrier-medium.

. In various embodiments the subject may be an animal such as a mammal including, human, horse, cow, dog, cat or any other animal in need of prevention and/or treatment of peripheral nerve damage.

. Various embodiments relate to a method of preventing and/or treating peripheral nerve damage comprising administering to a subject in need of preventing and/or treating peripheral nerve damage (a) a topical preparation comprising, less than or equal to 150 µM of 3,7-bis(dimethylamino) phenothiazin-5-ium chloride; and a physiological buffer or (b) a topical preparation as described herein above, or (c) a topical preparation comprising, less than or equal to 150 µM of 3,7-bis(dimethylamino) phenothiazin-5-ium chloride; and a carrier or carrier-medium.

. In various embodiments the topical preparation is an ophthalmic topical preparation.

. As used herein a subject refers to a patient or an individual diagnosed with, at high risk or having or suspected of having peripheral neuropathy. In various embodiments the patient or individual may be diagnosed with DM or pre-diabetes or suspected of having or at risk of developing DM or an individual with dry eye disease. In various embodiments the patient or individual may be diagnosed with neurotrophic keratopathy or at risk of developing neurotrophic keratopathy. In various embodiments the patient or individual may have peripheral neuropathy caused by traumatic injuries, infections, metabolic problems, inherited causes, ageing, exposure to toxins such as chemotherapy, hyperglycemia and/or hyperosmolarity induced peripheral nerve damage, or any other cause known in the art that results in peripheral nerve damage.

. In various embodiments the topical preparation is administered directly to the peripheral nerve site. In various embodiments the topical preparation is administered into the eye.

. In various embodiments the method further comprises the step of measuring parameters of corneal nerves to determine whether the subject is in need of preventing and/ treating peripheral nerve damage prior to administration of the topical preparation. In various embodiments the parameters measured comprise nerve fiber length, nerve fiber density. In various embodiments the parameters are measured with *in vivo* or *in situ* confocal microscopy of the cornea. The condition of corneal nerve fibers has been demonstrated to correlate to DM and peripheral neuropathy in other parts of a subject's body (Han et al. 2019).

. In various embodiments the method further comprises the step of measuring parameters of corneal nerves before and/or after administration of the topical preparation. In various embodiments the parameters measured comprise nerve fiber length, nerve fiber density. In various embodiments the parameters are measured with *in vivo* or *in situ* confocal microscopy of the cornea. This may allow monitoring of the effectiveness of the prevention and/or treatment.

. Various embodiments relate to the use of a diaminophenothiazine compound or tautomeric forms of formula I in the manufacture of a topical preparation for prevention and/or treatment of peripheral nerve damage.

. In various embodiments the compound is suitable for use in animals including humans and may be manufactured in a topical preparation suitable for prevention and/or treatment of peripheral nerve damage in medical or veterinary settings.

. In various embodiments the peripheral nerve damage results in or comprises neurotrophic keratopathy. In various embodiments the peripheral nerve damage comprises peripheral neuropathy caused by traumatic injuries, infections, autoimmune conditios, metabolic problems, inherited causes, ageing, exposure to toxins such as chemotherapy, hyperglycemia induced peripheral nerve damage, or any other cause known in the art that results in peripheral nerve damage.

. Various embodiments relate to the use of 3,7-bis(dimethylamino) phenothiazin-5-ium chloride in the manufacture of a topical preparation. comprising, less than or equal to 150 µM of 3,7-bis(dimethylamino) phenothiazin-5-ium chloride; and a physiological buffer or a carrier or carrier-medium for prevention and/or treatment of peripheral nerve damage.

. In various embodiments the topical preparation for prevention and/or treatment of peripheral nerve damage is as described herein.

. In various embodiments the use is for ophthalmic use. In various embodiments the use is for treatment of dry eye. In various embodiments the use is for prevention of corneal peripheral neuropathy.

. Various embodiments relate to a topical preparation for preventing and/or treating peripheral nerve damage comprising, less than or equal to 150 µM of 3,7-bis(dimethylamino) phenothiazin-5-ium chloride and a carrier or carrier-medium that adjusts or increases the viscosity of the preparation to a range of about 15 to 150 milli-Pascals per second.

. In various embodiments the topical preparation comprises a carrier or carrier - medium. In various embodiments the carrier or carrier-medium may comprise polymers such as hydrophilic polymers of high molecular weight, polyvinyl alcohol, poloxamers, hyaluronic acid, carbomers, cellulose derivatives (gellan gum, xanthan gum), 2-hydroxypropyl-beta-cyclodextrin, poly(2-hydroxyethylmethacrylate), ethylene glycol dimethylacrylate, acrylamide, N-vinylpryrrolidone and ethylacrylate or combinations thereof. In various embodiments the carrier or carrier- medium may comprise excipients that may increase drug delivery or penetration into the eye such as chelating agents, surfactants or cyclodextrins. In various embodiments the carrier or carrier -medium may comprise inclusion complexes such as nanoparticles, microparticles, niosomes, discosomes, dendrimers, in situ gels, wafers or embedded in contact lenses. In various embodiments the carrier or carrier-medium may comprise sugar molecules such as sucrose, glucose, lactose D-glucose (dextrose). In various embodiments the endogenous glucose naturally occurring in the peripheral nerve environment may form part of the carrier or carrier-medium in solution. This is particularly relevant in subjects with DM who have hyperglycemia involving a high concentration of glucose

. In various embodiments the carrier or carrier-medium modulates such as reduces the release profile of 3,7-bis(dimethylamino) phenothiazin-5-ium chloride. Any known carrier or carrier- medium that is able to modulate slow release profile such as any carrier or carrier - medium able to modulate such as reduce the rate of MTC release into the peripheral nerve environment would be suitable. In various embodiments the carrier or carrier-medium modulates such as reduces the release profile of 3,7-bis(dimethylamino) phenothiazin-5-ium chloride to a predetermined rate below or slower than the rate of release of 3,7-bis(dimethylamino) phenothiazin-5-ium chloride alone. In various embodiments a suitable carrier or carrier- medium may comprise polymers such as hydrophilic polymers of high molecular weight, polyvinyl alcohol, poloxamers, hyaluronic acid, carbomers, cellulose derivatives (gellan gum, xanthan gum), 2-hydroxypropyl-beta-cyclodextrin, poly(2-hydroxyethylmethacrylate), ethylene glycol dimethylacrylate, acrylamide, N-vinylpryrrolidone and ethylacrylate or combinations thereof. In various embodiments a suitable carrier or carrier-medium may comprise inclusion complexes such as nanoparticles, microparticles, niosomes, discosomes, dendrimers, in situ gels, wafers or may be embedded in contact lenses.

. In various embodiments the carrier or carrier-medium adjusts such as increases the viscosity to a range of about 15 to 150 milli-Pascals per second. In various embodiments the carrier or carrier-medium adjusts such as increases the viscosity to a range of about 15 to 100 milli-Pascals per second. In various embodiments the carrier or carrier-medium adjusts such as increases the viscosity to a range of about 15 to 50 milli-Pascals per second. In various embodiments the viscosities are those at room temperature. Increased viscosity may have the advantage of enabling longer contact time between MTC and the corneal surface, thus increasing its bioavailability. In various embodiments the polymers, sugar molecules, nanoparticles, microparticles, niosomes, discosomes, dendrimers carriers or carrier-medium may adjust or increase the viscosity of the solution.

. In various embodiments the carrier or carrier-medium modulates such as reduces the release profile of 3,7-bis(dimethylamino) phenothiazin-5-ium chloride. In various embodiments the carrier or carrier-medium comprises a micro-particle with a slow release profile. This has the advantage of increasing the viscosity of the preparation and it also reduces the need for frequent applications of eye drops translating to better patient compliance. The slow release formulation also has the advantage of reducing unsightly blue stains on the skin as the increased viscosity reduces dripping and/or release with tears causing less stains and discoloration on the face. In various embodiments the carrier or carrier-medium micro-particle with a sustained/slow release profile comprises a poly (lactide-co-glycolide) (PLGA) microspheres however any microparticle known in the art to be able to provide a sustained/slow release profile would be suitable. In various embodiments the PLGA microspheres comprise a polymer ratio of PLGA (50:50), PLGA (75:25), or PLGA (85:15). Such ratios may be able to further modulate such as reduce the release profile of MTC. In various embodiments the release profile of MTC may be modulated to 1, 3, or 6 months with the use of different polymer ratios in the microspheres and size of the microspheres.

. In various embodiments the carrier or carrier-medium comprises a microparticle as described herein.

. In various embodiments the carrier or the carrier-medium comprises an aqueous fluid medium such as a gel.

. In various embodiments the carrier or carrier-medium comprises a gel as described herein.

. As used herein the term 'gel' may refer to a cross linked network comprising a 3 dimensional semi-solid matrix containing or able to contain a high percentage of liquid content.

. Topical Methylene blue has generally never been shown to be efficacious in PNS disorders. The devised topical preparation has an indication to manage peripheral neuropathy that may be caused by traumatic injuries, infections,autoimmune conditions, metabolic problems, inherited causes, ageing, exposure to toxins such as chemotherapy, hyperglycemia induced peripheral nerve damage, or any other cause known in the art that results in peripheral nerve damage. This can be preventive and/or therapeutic.

. Various embodiments relate to a topical kit comprising a) a topical composition comprising of an effective amount of diaminophenothiazine such as 3,7-bis(dimethylamino) phenothiazin-5-ium chloride , and b) a container for holding the said composition for use in a method of preventing and/or treating peripheral nerve damage and its associated conditions. In various embodiments peripheral nerve damage and its associated conditions may comprise peripheral nerve damage of the eye relating to corneal nerve injury and its associated conditions including neurotrophic keratopathy.

. In various embodiments, the container is configured to hold an amount of the said topical composition supporting a treatment for at least 1, 7,14, 21 or 30 days.

. In various embodiments, the kit comprises an eye drop kit. In various embodiments, the container may comprises a breakable seal.

. Various embodiments relate to a method of formulating a topical composition comprising: encapsulating 3,7-bis(dimethylamino) phenothiazin-5-ium chloride in a microparticle or any other carrier-medium capable of slow release profile.

. In various embodiments the microparticle or carrier-medium capable of slow release profile further comprises a fluid or aqueous medium.

. In various embodiments the carrier-medium is capable of carrying more than 1 compound.

. In various embodiments, the method of formulating the topical composition comprises fulfilling at least some of the following conditions: a) do not contain any part/s or product/s of animal/s that are non-halal by Shariah law or any part/s or product/s of animal/s which are not slaughtered according to Shariah law; b) do not contain najs according to Shariah law; c) safe for human use non-poisonous, non-intoxicating or non-hazardous to health according to prescribed dosage; d) not prepared, processed or manufactured using equipment contaminated with najs according to Shariah law; e) do not contain any human part/s or its derivatives that are not permitted by Shariah law; and f) during its preparation, processing, handling, packaging, storage and distribution, the halal pharmaceutical product/s are physically separated from any other pharmaceutical product/s that do not meet the requirement/s stated in item/s a), b), c), d) or e) or any other item/s that have been decreed as non-Halal and najs by Shariah law.

. Throughout the specification, unless otherwise indicated to the contrary, the terms "comprising", "consisting of", and the like, are to be construed as non-exhaustive, or in other words, as meaning "including, but not limited to".

. Throughout the specification, unless the context requires otherwise, the word "comprise" or variations such as "comprises" or "comprising", will be understood to imply the inclusion of a stated integer or group of integers but not the exclusion of any other integer or group of integers.

. Throughout the specification, unless the context requires otherwise, the word "include" or variations such as "includes" or "including", will be understood to imply the inclusion of a stated integer or group of integers but not the exclusion of any other integer or group of integers.

. As used herein, the term "about" typically means +/- 5% of the stated value, more typically +/- 4% of the stated value, more typically +/- 3% of the stated value, more typically +/- 2% of the stated value, even more typically +/- 1% of the stated value, and even more typically +/- 0.5% of the stated value.

. Unless defined otherwise, all technical and scientific terms used herein have the same meaning as is commonly understood by a skilled person to which the subject matter herein belongs. As used herein, the following definitions are supplied in order to facilitate the understanding of the present invention.

. Throughout this document, unless otherwise indicated to the contrary, the terms "comprising", "consisting of", "having" and the like, are to be construed as non-exhaustive, or in other words, as meaning "including, but not limited to".

. Furthermore, throughout the specification, unless the contest requires otherwise, the word "include" or variations such as "includes" or "including" will be understood to imply the inclusion of a stated integer or group of integers but not the exclusion of any other integer or group of integers.

. As used in the specification and the appended claims, the singular form "a", and "the" include plural references unless the context clearly dictates otherwise.

### . Examples.

### . Example 1: Prevention of damage of peripheral nerves by glucose.

. In-vitro cell assay based experiment demonstrated the ability for methylene blue to protect the dorsal root ganglion neurones and its nerve extensions from the insult of high glucose concentration with just 1 hour of pre-insult incubation.

. The objective of the present study is to explore the dose-dependent neuroprotective effects of methylene blue (MB) against the neurotoxicity induced by high glucose level using a multi-parametric cell-based protocol that reflects the mechanisms of action. The assay was carried out using freshly isolated rat dorsal root ganglion neurons (DRG) cultured in 96-well-plates pre-treated with different concentrations of MTC compound (0.0005 µM, 0.005 µM, 0.05 µM, 0.5 µM, 5 µM and 50 µM) prior to exposure to 150 mM glucose for 48 hours.

. In vitro injury was induced in DRGs culture by glucose treatment (150 mM) for 48 hours. DRGs were incubated with six increasing concentrations of the test compound (0.0005 µM, 0.005 µM, 0.05 µM, 0.5 µM, 5 µM and 50 µM) for 1 hour prior to the glucose treatment followed by 48 hrs of co-incubation with high dose glucose. After treatments, cells were fixed and stained with anti-beta III tubulin, and were then analyzed with BD Pathway 855 (Becton Dickinson). By using HCS technology, cell parameters associated with neurite outgrowth - an indication of pre-lethal cytotoxic effects - were measured at single cell level, which allows high-throughput screening.

. In order to compare the degree of neuroprotection of the compound at different concentrations, the level of neuroprotection of each concentration of the compound was studied and four different scores of neuroprotection were established according to the level of variation when compared with control cells: No neuroprotection, low neuroprotection, moderate neuroprotection, and high neuroprotection.

. Primary cultures of dorsal rat ganglion neurons were prepared from the dorsal ganglions of neonatal Sprague-Dawley rats at day 8. Ganglions were removed from spine under a binocular microscope and neurons were enzymatically dispersed twice with collagenase I (Worthington) 1.25 mg/ml during 1 hour at 37°C and plated on poly-L-lysine (Sigma Aldrich # Cat.P1524) wells with neurobasal medium (Invitrogen # Cat.21103-049) supplemented with B27(Invitrogen # Cat. 17504-044) with a number of 30,000 cells per well. Cells were maintained in neurobasal medium supplemented with B-27 component for 8 days at 37°C in a humidified 5% CO₂ atmosphere.

. Methylene blue compound (Tocris) was solubilized in ethanol to a final concentration of 10mM. The concentration of the test compound was then adjusted in neurobasal medium (Invitrogen # Cat.21103-049) supplemented with 20% B27 (Invitrogen # Cat. 17504-044).

. At day 8, cells were pre-treated for 1 hour with 6 increasing concentrations of methylene blue (0.0005 µM, 0.005 µM, 0.05 µM, 0.5 µM, 5 µM, and 50 µM) in complete medium. Then, medium was replaced with neurobasal medium without the B27 component (80%) plus neurobasal medium supplemented with B27 component (20%) for 48h in the presence of glucose 150 mM with Methylene Blue co-incubated at different concentrations. See figure 1 for the testing timeline.

. The neurobasal medium are basal media formulated to meet the neuronal cells special requirements. It allows for long-term maintenance of the normal phenotype and growth of neuronal cells, and maintain pure populations of neuronal cells without the need of an astrocyte feeder layer serum free. The use of the medium results in a methylene blue solution with a higher pH in the range of 4 to 8 which is suitable for use with neuronal cells. The solution increases the viscosity.

| **Components of neurobasal medium** | **Molecular Weight** | **Concentration (mg/L)** | **mM** |
|---|---|---|---|
| Amino Acids | | | |
| Glycine | 75.0 | 30.0 | 0.4 |
| L-Alanine | 89.0 | 2.0 | 0.02247191 |
| L-Arginine hydrochloride | 211.0 | 84.0 | 0.39810428 |
| L-Asparagine-H₂O | 150.0 | 0.83 | 0.0055333334 |
| L-Cysteine | 121.0 | 31.5 | 0.2603306 |
| L-Histidine hydrochloride-H2O | 210.0 | 42.0 | 0.2 |
| L-Isoleucine | 131.0 | 105.0 | 0.8015267 |
| L-Leucine | 131.0 | 105.0 | 0.8015267 |
| L-Lysine hydrochloride | 183.0 | 146.0 | 0.7978142 |
| L-Methionine | 149.0 | 30.0 | 0.20134228 |
| L-Phenylalanine | 165.0 | 66.0 | 0.4 |
| L-Proline | 115.0 | 7.76 | 0.06747826 |
| L-Serine | 105.0 | 42.0 | 0.4 |
| L-Threonine | 119.0 | 95.0 | 0.79831934 |
| L-Tryptophan | 204.0 | 16.0 | 0.078431375 |
| L-Tyrosine | 181.0 | 72.0 | 0.39779004 |
| L-Valine | 117.0 | 94.0 | 0.8034188 |

| **Vitamins** | | | |
|---|---|---|---|
| Choline chloride | 140.0 | 4.0 | 0.028571429 |
| D-Calcium pantothenate | 477.0 | 4.0 | 0.008385744 |
| Folic Acid | 441.0 | 4.0 | 0.009070295 |
| Niacinamide | 122.0 | 4.0 | 0.032786883 |
| Pyridoxal hydrochloride | 204.0 | 4.0 | 0.019607844 |
| Riboflavin | 376.0 | 0.4 | 0.0010638298 |
| Thiamine hydrochloride | 337.0 | 4.0 | 0.011869436 |
| Vitamin B12 | 1355.0 | 0.0068 | 5.0184503E-6 |
| i-Inositol | 180.0 | 7.2 | 0.04 |

| **Inorganic Salts** | | | |
|---|---|---|---|
| Calcium Chloride (CaCl₂) (anhyd.) | 111.0 | 200.0 | 1.8018018 |
| Ferric Nitrate (Fe(NO₃)_{3*}9H₂O) | 404.0 | 0.1 | 2.4752476E-4 |
| Magnesium Chloride (anhydrous) | 95.0 | 77.3 | 0.8136842 |
| Potassium Chloride (KCl) | 75.0 | 400.0 | 5.3333335 |
| Sodium Bicarbonate (NaHCO₃) | 84.0 | 2200.0 | 26.190475 |
| Sodium Chloride (NaCl) | 58.0 | 3000.0 | 51.724136 |
| Sodium Phosphate monobasic (NaH₂PO₄-H₂O) | 138.0 | 125.0 | 0.9057971 |
| Zinc sulfate (ZnSO₄*7H₂O) | 288.0 | 0.194 | 6.736111E-4 |

| **Other Components** | | | |
|---|---|---|---|
| D-Glucose (Dextrose) | 180.0 | 4500.0 | 25.0 |
| HEPES | 238.0 | 2600.0 | 10.92437 |
| Phenol Red | 376.4 | 8.1 | 0.021519661 |
| Sodium Pyruvate | 110.0 | 25.0 | 0.22727273 |

. **High Content Screening assay:** Methylene blue (MB) neuroprotection was determined by high content screening (HCS) analysis, which includes the following endpoints: neurite outgrowth, cell number, and cell viability.
1. Cell number: For determining cell number, hoechst 33342 nucleic acid stain was used. Cells were stained with 5 µg/ml hoechst 33342 (Invitrogen # Cat.H1399), washed 3 times and with fluorescence measured at an excitation wavelength of 380 nm and emission wavelength of460 nm. This dye allows a sensitive cell number determination by fluorescence microscopy.

. Glucose treatment resulted in 54% of mortality in neurons after exposure at 150 mM concentration and methylene blue co-incubation could prevent glucose-induced mortality in all concentrations in a dose dependent manner with the exception of the highest concentration (50 µM). Moreover, it was also observed that this concentration of the compound in addition to glucose induced an additional increase in mortality (figure 2 and table 1).

**Table 1: data of HOECHST analysis. Values represent the mean at each condition (triplicates average).**

| DAPI | Cell viability by Hoechst | | | mean | Standard deviation | | Mean % of control | Standard deviation |
|---|---|---|---|---|---|---|---|---|
| Control | 223 | 224 | 218 | 221.666667 | 3.21455025 | Control | 100 | 1.4545476 |
| Glucose (Alone) | 125 | 104 | 81 | 103.333333 | 22.0075745 | Glucose (Alone) | 46.757164 4 | 9.9581784 |
| MB 0.0005 uM (with glu) | 187 | 236 | 242 | 221.666667 | 30.1717307 | MB 0.0005 uM (with glu) | 100.30165 9 | 13.652366 |
| MB 0.005 uM (with glu) | 255 | 290 | 218 | 254.333333 | 36.0046293 | MB 0.005 uM (with glu) | 115.08295 6 | 16.291687 |
| MB 0.05 uM (with glu) | 270 | 253 | 241 | 254.666667 | 14.571662 | MB 0.05 uM (with glu) | 115.23378 6 | 6.5935122 |
| MB 0.5 uM (with glu) | 234 | 217 | 273 | 241.333333 | 28.7112057 | MB 0.5 uM (with glu) | 109.20060 3 | 12.991495 |
| MB 5 uM (with glu) | 168 | 103 | 157 | 142.666667 | 34.7898453 | MB 5 uM (with glu) | 64.555052 8 | 15.742011 |
| MB 50 uM (with glu) | 74 | 69 | 62 | 68.3333333 | 6.02771377 | MB 50 uM (with glu) | 30.920060 3 | 2.7274723 |

. Different grades of neuroprotection were observed with the rest of the concentrations tested therefore, methylene blue supplementation significantly reduced cell death and improved survival of neurons and nerve projections.

. 2. WST-8 assay: For detection of viable cells, 10 µl of CCK -8 reagent (WST-8) were added to each well and the plate was incubated at 37°C. After 1 hour, absorbance was measured at 450 nm using the Synergy II microplate reader.

. Glucose treatment resulted in 22% of viability reduction in DRGs when measured by WST8 after exposure but the compound could not prevent glucose induced viability decrease (figure 5). It was also observed that the highest concentrations of the compound (50 µM) in addition to glucose induced a decrease in viability.

. 3. Beta-III tubulin staining: Beta-III tubulin staining was determined by Immunohistochemistry (IHQ). Cells were washed with PBS (Sigma Aldrich # Cat.D8537) and fixed with methanol for 10 minutes. After the fixation step the samples were washed three times with PBS and permeabilized with PBS + 0.3% triton for 10 minutes. The samples were then blocked with PBS + Bovine Serum Albumine (BSA) for 30 minutes and finally anti-beta tubulin III antibody (Abcam # Cat.ab7751) were added at 1/1000 in PBS + 0.5% BSA for 60 minutes at room temperature. After three washing steps, the secondary antibodies, anti-goat-antimouse Alexa 633 (Abcam # Cat.A21050) were added at 1/100 for 60 minutes to react against the primary antibody. The samples were then washed three times and measured under automated fluorescent microscope. To investigate the role of neurite extension, two geometric patterns were mainly employed in this study: total neurite length (total length of the path from a neuron body), and number of extremities (number of terminating neurite segments per neuron).

. At the end of the assay, for measuring viable cells, cells were firstly stained with WST-8 for 1 hour and then fixed with methanol for beta-III tubulin staining and imaged again using BD Pathway 855. In order to acquire enough cells for the analysis, nine fields per well were imaged. The 20x objective was used to collect images for the distinct fluorescence channels. Dyes were excited and their fluorescence was monitored at the excitation and emission wavelengths with appropriate filter settings. In the setting up of the procedure, exposure times were adjusted in order to avoid overlapping emission between different probes. The collected images were analyzed using a module that allows the simultaneous quantification of subcellular structures, which are stained by different molecular probes to measure the fluorescence intensity associated with predefined nuclear and cytoplasmic compartments.

. To investigate the role of neurite extension two geometric patterns was mainly employed in this study; neurite total length per neuron and extremity count/neuron. Glucose treatment result in a decrease of 38% and 31% in neurite total length and neurite extremity count respectively compared to untreated cells, and on the other hand, methylene blue could re-establish the affected neurite outgrowth induced by glucose with 0.05 µM concentration (figure 3, 4, Tables 2 and 3). Therefore, methylene blue supplementation significantly increased neurite outgrowth and improved survival of neurons.

**Table 2: data of extremity count/neuron. Values represent the mean at each condition (triplicates average).**

| | Extremity count | | | mean | Standard deviation | | Mean % of control | Standa rd deviati on |
|---|---|---|---|---|---|---|---|---|
| Control | 1.143497 76 | 1.28125 | 1.311926 61 | 1.245558 12 | 0.089707 87 | Control | 100 | 7.2345 056 |
| Glucose (Alone) | 0.82 | 0.986206 9 | 0.781818 18 | 0.862675 03 | 0.108671 78 | Glucose (Alone) | 69.57056 66 | 8.7638 5297 |
| MB 0.0005 uM (with glu) | 1.058394 16 | 0.861290 32 | 0.978362 57 | 0.966015 69 | 0.099130 29 | MB 0.0005 uM (with glu) | 77.90449 08 | 7.9943 7846 |
| MB 0.005 uM (with glu) | 0.864516 13 | 1.189655 17 | 1.005031 45 | 1.019734 25 | 0.163067 41 | MB 0.005 uM (with glu) | 82.23663 3 | 13.150 5972 |
| MB 0.05 uM (with glu) | 1.344680 85 | 1.059405 94 | 1.140762 46 | 1.181616 42 | 0.146959 95 | MB 0.05 uM (with glu) | 95.29164 66 | 11.851 6091 |
| MB 0.5 uM (with glu) | 0.656716 42 | 0.855205 05 | 0.778388 28 | 0.763436 58 | 0.100085 46 | MB 0.5 uM (with glu) | 61.56746 62 | 8.0714 0792 |
| MB 5 uM (with glu) | 0.855882 35 | 0.536945 81 | 0.577707 01 | 0.656845 06 | 0.173572 04 | MB 5 uM (with glu) | 52.97137 56 | 13.997 745 |
| MB 50 uM (with glu) | 0.283333 33 | 0.234482 76 | 0.2625 | 0.260105 36 | 0.024513 17 | MB 50 uM (with glu) | 20.97623 9 | 1.9768 6833 |

**Table 3: data of total length analysis. Values represent the mean at each condition (triplicates average).**

| | Total length | | | mean | Standard deviation | | Mean % of control | Standard deviation |
|---|---|---|---|---|---|---|---|---|
| Control | 65.95818 97 | 71.38207 15 | 74.91194 5 | 70.75073 54 | 4.510141 07 | Control | 100 | 6.3747577 |
| Glucose (Alone) | 42.94953 52 | 52.96927 37 | 37.56238 44 | 44.49373 11 | 7.818661 33 | Glucose Alone) | 62.88866 59 | 11.051111 4 |
| MB 0.0005 uM (with glu) | 50.95711 79 | 40.13092 87 | 59.35768 | 50.14857 55 | 9.638843 17 | MB 0.0005 uM (with glu) | 70.88137 89 | 13.623806 6 |
| MB 0.005 uM (with glu) | 45.83479 2 | 61.19289 46 | 50.50447 19 | 52.51071 95 | 7.873157 01 | MB 0.005 uM (with glu) | 74.22009 82 | 11.128137 1 |
| MB 0.05 uM (with glu) | 62.91126 39 | 50.38960 69 | 69.80080 52 | 61.03389 2 | 9.840835 78 | MB 0.05 uM (with glu) | 86.26698 52 | 13.909308 5 |
| MB 0.5 uM (with glu) | 37.33583 24 | 47.76891 48 | 34.06626 92 | 39.72367 21 | 7.156602 39 | MB 0.5 uM (with glu) | 56.14653 3 | 10.115339 1 |
| MB 5 uM (with glu) | 53.61185 27 | 42.20316 16 | 32.29649 71 | 42.70383 71 | 10.66649 44 | MB 5 uM (with glu) | 60.35878 04 | 15.076317 2 |
| MB 50 uM (with glu) | 18.18108 85 | 12.64229 43 | 17.45257 66 | 16.09198 65 | 3.009645 23 | MB 50 uM (with glu) | 22.74485 72 | 4.2539155 1 |

. It was also observed that the highest concentrations of the compound (5 and 50 µM) in addition to glucose induced neurite outgrowth reduction.

. For all the studied parameters, a variation of at least 20% in fluorescence intensity or in the corresponding morphological parameter in relation to untreated cultures was considered. In order to compare the degree of neuroprotection of methylene blue, four different scores of neuroprotection were established according to the level of variation when compared with control cells: 0 (no neuroprotection or variation lower than 20%), 1 (variation 20-40%), 2 (variation 40-60%), 3 (variation 60-100%), 4 (variation 100-200%). Moreover, some concentrations presented certain degree of injury, maybe because they are toxic or maybe because they are toxic in combination with glucose, and similar scores of injury were established according to the level of variation when compared to glucose-treated cells: -1 (variation 20-40%), -2 (variation 40-60%) and -3 (variation 40- 60%). The sum of each individual score resulted in the total level of neuroprotection for each compound and was defined as its degree of neuroprotection. From this calculation, a neuroprotective scale was established: high (>6), moderate (4-5), low (1-3), and no neuroprotection (0).

. In order to compare the degree of neuroprotection of the tested compound at different concentrations, different scores were created according to the level of the alteration (the group formation criterion is described in material and methods section) and then a value was assigned to each parameter. The sum of all the values for each parameter and concentration tested was calculated, and four distinct groups were created according to their level of neuroprotection. From this analysis, the degree of neuroprotection of each compound studied was defined (table 4).

**Table 4. Degree of neuroprotection of methylene blue after analyzing data for each concentration in glucose-treated DRGs. The score of neuroprotection was establish as described in material and methods. The boxes highlighted in red represent toxicity.**

| | **0.0005 µM** | **0.005 µM** | **0.05 µM** | **0.5 µM** | **5 µM** | **50 µM** |
|---|---|---|---|---|---|---|
| **HOECHST** | 3 | 3 | 3 | 3 | 1 | -1 |
| **Extremity count** | 0 | 0 | 1 | 0 | -1 | -3 |
| **Total length** | 0 | 0 | 1 | 0 | 0 | -1 |
| **WST8** | 0 | 0 | 0 | 0 | 0 | -1 |
| **Total** | 3 | 3 | 5 | 3 | 0 | -6 |
| **Neuroprotection degree** | low | low | moderate | low | no | toxic |

. Briefly, methylene blue scored moderate neuroprotection at 0.05 µM, low neuroprotection at 0.0005 µM, 0.005 µM, and 0.5 µM, no neuroprotection at 5 µM and toxicity at 50 µM.

. The neuroprotection effect of methylene blue is shown in the table 5 below:

**Table 5: Classification of the degree of neuroprotection provided by different concentrations of methylene blue prior to assault with 150 mM glucose.**

| | **0.0005 µM** | **0.005 µM** | **0.05 µM** | **0.5 µM** | **5µM** | **50 µM** |
|---|---|---|---|---|---|---|
| **Neuroprotection degree** | low | low | moderate | low | no | no Toxic |

. According to all parameters studied, methylene blue showed some neuroprotection against glucose-induced DRGs toxicity at concentrations from 0.0005 µM to 0.5 µM and moderate neuroprotection effect against glucose-induced DRGs toxicity at 0.05 µM.

. In the present study, glucose toxicity resulted in a decrease in neurite outgrowth and cell number and viability. The preventive effects of methylene blue against glucose toxicity is associated with increase of cell viability and restoration of neurite outgrowth. Attending to all parameters studied, methylene blue supplementation scores moderate neuroprotection levels at 0.05 µM.

### . Example 2: Testing of topical Methylene blue preparation tolerance in the human eye (in vivo)

. To establish the concentrations of Methylene blue topical preparations that can be used in the human eye, including and up to 0.005 % (160µM), a series of topical ophthalmic preparations concentrations were prepared and tested. Methylene blue compound (Bleu de Methylene 1%, STEROP) was solubilized in sterile lubricant eye gel (Systane Gel Drop) to a volume of 10 mls to a final maximum concentration of 160microM. The concentration of the topical preparations was adjusted to 0.05µM, 0.5µM, 5µM, 50µM, 60µM, 150µM and 160µM. The viscosity of the eye drops were increased with the use of the lubricant eye gel (Systane Gel Drop). One drop of each of the topical preparations was applied to each human eye once per day. The eyes were examined immediately after application, at 6 hours and 24 hrs after application using standard optometry scan and displayed no adverse effects at all the time points.

. In all the topical preparations including at 150µM and below the methylene blue topical preparations were observed to be fully tolerable to the eye without causing any damage nor discomfort. Above 150µM, it gives a slight stingy sensation to the eye causing temporary mild discomfort which is self-limiting in nature. The viscosity of this gel-like topical preparation allows the topical preparations to reside within the eye and not streak down the eyelid onto the face. This "non drip" effect is very desirable.

### . Example 3: Formation of topical preparations

### . Preparation A

. Methylene blue compound (Tocris) will be solubilized in sterile water to a final concentration of 10mM. The concentration of the test compound will be adjusted to 0.05µM, 0.5µM, 5µM, 50µM, or 100µM in 10mM HEPES buffer containing sodium bicarbonate and sodium chloride.

### . Preparation B

. Fluid gels will be produced by first dissolving low acyl gellan gum (Kelco gel CG LA, Azelis, UK) in deionized water. Gellan powder will be added to deionized water at ambient temperature in the correct ratio to result in a 1% (w/v) solution. The sol will be heated to 70 °C under agitation, on a hotplate equipped with a magnetic stirrer, until all the polymer has dissolved. Once dissolved, gellan sol will be added to the cup of a rotational rheometer equipped with cup and vane geometry (cup: 35mm diameter, vane: 28mm diameter). The system will then be cooled to 40 °C. Methylene blue compound (Tocris) will be solubilized in sterile water to a final concentration of 10mM. The concentration of the test compound will be adjusted to 0.05µM, 0.5µM, 5µM, 50µM, or 100µM in phosphate-buffered saline (PBS) (4.76 mg/ml) and aqueous sodium chloride (0.2 M). Following this, the mixture will be cooled at a rate of 1 °C/min under shear (450/s) to a final temperature of 20 °C. The sample will then be removed and stored at 4 °C until further use. In the case of fluid gels without Methylene blue compound, ratios will be adjusted so that the final eye drop has a composition of 0.9% (w/v) gellan, 10mM NaCl.

### . Preparation C

. Encapsulation of PLGA requires a compound with high solubility in DI water. The solubility of MTC was therefore tested. 20 ml of solvent was placed in a glass vial and magnetic stirrer. methylene blue, 88.0% purity (Merck) was added until saturation point, or in excess of. Stirring was performed for period length, as necessary for slow-dissolution methylene blue, 88.0% purity (Merck). The amount of methylene blue, 88.0% purity (Merck) dissolved was measured to obtain the solubility of the methylene blue, 88.0% purity (Merck) in the specific solvent.

. Results of Solubility of methylene blue, 88.0% purity (Merck) in:
.
.
.

| | |
|---|---|
| DI Water: | 41.432 mg/ml |
| Dichloromethane (DCM): | < 0.1 mg/ml |
| Acetonitrile (ACN): | 0.5675 mg/ml |

. Findings of the solubility of methylene blue suggest initial incompatibility with the PLGA carrier, though final outcome is successful surprisingly.

### . Encapsulation

. Production of the microspheres was performed as follows over 3 separate runs on three occasions. The case for methylene blue is unique; the dye is distinct in colour and visual inspection may be done to confirm loading of the methylene blue into the PLGA microspheres. The 3 production runs were conducted to optimize the loading efficiency of the methylene blue into the microspheres.

. Oil in Water emulsions was generated using a glass capillary microfluidic setup. The axisymmetric coaxial glass capillary flow-focusing device was assembled using a square and round capillary. The surface of round capillary was hydrophilized with the treatment of oxygen plasma (100 Watts) for 120 seconds. The aqueous continuous phase (W) was prepared by mixing PVA (1 % w/v) in water. The dispersed phase (O) was conducted with methylene blue, 88.0% purity (Merck). One of the following concentrations of methylene blue, 88.0% purity (Merck) was used i) 0.5µM, ii) 50µM, 150µM respectively). PLGA concentration in dichloromethane (DCM) (50 mg per mL) was kept constant for all the three cases. W and O phases was infused from the two ends of the square capillary through the outer coaxial region using syringe pumps (Harvard PHD 22/2000 series) at flow rates of 150 and 50 µL per min respectively. The fluids were hydro-dynamically flow focused through the nozzle of the round capillary resulting in the formation of emulsion droplets. 6 cm ID glass wells were used for sample collection. Approximately 300 µL of O/W emulsions was dispensed directly into a glass well containing a predispensed film (3 mL) of continuous phase to prevent droplet coalescence. Solvent evaporation was performed at room temperature (24 °C). Uniform microspheres were collected encapsulating methylene blue validated using microscope imaging.

### . Characterization of the microspheres

. Microsphere sizes ranging 10 - 25 µm is optimal to maximize the performance of PLGA microspheres. Characterization was performed by measuring 100 microspheres using the software ImageJ and reporting the average and standard deviation.

### . Results

. The sizes of microspheres manufactured from each production run are as follows:
.
.
.

| | |
|---|---|
| Run 1: | 25.45 ± 1.13 µm |
| Run 2: | 20.46 ± 1.12 µm |
| Run 3: | 16.69 ± 0.74 µm |

. The micrographs of each run are depicted in figures 6, 7 and 8. Runs 2 and 3 meet the acceptance criteria.

### . Example 4: Release profile of preparation C

. 5 mg of the dried microspheres manufactured from Run 2 as described above was used for a 7-day release study. The following time points were measured to evaluate the release profile: 0, 3, 6, 9, 12, 24, 48, 72, 96, 120, 144 and 168 hours.

. A small sample of the dried microspheres would also be measured to evaluate the amount of methylene blue loading.

. HPLC was used to determine the concentrations in each sample.

### . Results

. Results from the 7-day methylene blue release from the microspheres produced in Run 2 are as follows:

**Table 6: Mass of methylene blue released from PLGA microspheres from production Run 2 in 7 days.**

| **Time (h)** | **Methylene blue released (µg)** | **Cumulative release (%)** |
|---|---|---|
| 0 | 0.086 | 1.92 |
| 3 | 0.611 | 13.55 |
| 6 | 0.955 | 21.20 |
| 9 | 1.215 | 26.97 |
| 12 | 1.453 | 32.25 |
| 24 | 2.075 | 46.06 |
| 48 | 2.907 | 64.52 |
| 72 | 3.530 | 78.32 |
| 96 | 3.885 | 86.20 |
| 120 | 4.226 | 93.77 |
| 144 | 4.369 | 96.95 |
| 168 | 4.506 | 100.00 |

. Successful microsphere production with methylene blue encapsulation within the PLGA microspheres target size range was achieved. The results were confirmed by the 7-day release study showing steady release of methylene blue. Microspheres were coated with a mucoadhesive coating.

. The encapsulation resulted in substantial methylene blue loading into the PLGA microspheres in the production runs. This was confirmed using the HPLC. The release profile is depicted in Table 6 and Figure 9.

### . Example 5: In vivo prevention and/or treatment of peripheral nerve damage of the eye.

. Methylene blue compound (Tocris) will be solubilized in sterile water to a final concentration of 10mM. The concentration of the test compound will be adjusted to 0.05µM, 0.5µM, 5µM, 50µM, or 100µM in 10mM HEPES buffer containing sodium bicarbonate and sodium chloride (preparation A). An untreated group only contained 10mM HEPES buffer containing sodium bicarbonate and sodium chloride. A second group of the same test solutions will be made with the viscosity adjusted to be increased with 5g/l gellan gum (preparation B). A third group of the same test solutions will be made with the viscosity adjusted to be increased using a slow release micro-particle (preparation C).

. The solutions will be dropped in a diabetic mouse eyes once daily for 2 weeks. In-vivo Confocal Microscopy will be performed using a modified Heidelberg Retina Tomograph Rostock Corneal Module. Before scanning, mice were anesthetized with a single injection of 50 mg/kg ketamine and 5 mg/kg xylazine and one drop of topical proparacaine 0.5% ophthalmic solution (Bausch & Lomb, Tampa, FL). Lubrication was maintained on the contralateral eye using Refresh Plus (Allergan, Irvine, CA). To enhance epithelial viewing, a silicon washer was placed on the outer edge of the TomoCap (Heidelberg, Germany) to generate a thin layer of space between the TomoCap tip and the surface epithelium.17 The dimensions of the washer were as follows: 1.2-cm outer diameter, 3-mm inner diameter, and 600-mm thick (Specialty Silicone Products, Inc., Ballston Spa, NY). Image acquisition was performed as previously described.17 Corneas were scanned at a lens speed of 30 mm per second with 1-mm increment steps. A minimum of three scans was performed on each cornea.

. The repeated treatment is envisioned to increase the effectiveness of the topical methylene blue ophthalmic preparations in a dose-dependent manner, and the increase viscosity of the solution at 5µM, 50µM, and 100µM is envisioned to increase the effectiveness of the methylene blue ophthalmic solution.

. It should be further appreciated by the person skilled in the art that variations and combinations of features described above, not being alternatives or substitutes, may be combined to form yet further embodiments falling within the intended scope of the invention.

. As would be understood by a person skilled in the art, each embodiment, may be used in combination with other embodiment or several embodiments.

## Claims

1. A topical preparation for use in preventing and/or treating peripheral nerve damage on body surfaces including mucous membranes or a nerve site on the surface of the cornea comprising an effective amount of 3,7-bis(dimethylamino) phenothiazin-5-ium chloride and a carrier-medium comprising (i) a microparticle with a sustained release profile of 3,7-bis(dimethylamino) phenothiazin-5-ium chloride, wherein the microparticle comprises poly (lactide-co-glycolide); or (ii) a polymer selected from hydrophilic polymers of high molecular weight, polyvinyl alcohol, poloxamers, hyaluronic acid, carbomers, cellulose derivatives (gellan gum, xanthan gum), 2-hydroxypropyl-beta-cyclodextrin, poly(2-hydroxyethylmethacrylate), ethylene glycol dimethylacrylate, acrylamide, N-vinylpryrrolidone and ethylacrylate or combinations thereof.

2. The topical preparation for use according to claim 1, wherein the topical preparation comprises between 0.0001 µM and 60 µM of 3,7-bis(dimethylamino) phenothiazin-5-ium chloride or wherein the topical preparation comprises 0.0005 µM, 0.005 µM, 0.05 µM, 0.5 µM, 5 µM, 50µM, or 100µM of 3,7-bis(dimethylamino) phenothiazin-5-ium chloride.

3. The topical preparation for use according to claim 1, wherein the topical preparation comprises less than or equal to 150 µM 3,7-bis(dimethylamino) phenothiazin-5-ium chloride.

4. The topical preparation for use according to any one of claims 1 to 3, further comprising a physiological buffer, wherein the physiological buffer comprises
a) sodium bicarbonate and sodium chloride;
b) 4-(2-hydroxyethyl)-1-piperazineethanesulfonic acid; or
c) a neurobasal medium selected from the group consisting of Glycine, L-Alanine, L-Arginine hydrochloride, L-Asparagine-H₂O, L-Cysteine, L-Histidine hydrochloride-H₂O, L-Isoleucine, L-Leucine, L-Lysine hydrochloride, L-Methionine, L-Phenylalanine, L-Proline, L-Serine, L-Threonine, L-Tryptophan, L-Tyrosine, L-Valine, Choline chloride, D-Calcium pantothenate, Folic Acid, Niacinamide, Pyridoxal hydrochloride, Riboflavin, Thiamine hydrochloride, Vitamin B12, i-Inositol, Calcium Chloride (CaCl₂) (anhyd.), Ferric Nitrate (Fe(NO₃) *9H₂O), Magnesium Chloride (anhydrous), Potassium Chloride (KCI), Sodium Bicarbonate (NaHCO₃), Sodium Chloride (NaCl), Sodium Phosphate monobasic (NaH₂PO₄-H₂O), Zinc sulfate (ZnSO₄*7H₂O), D-Glucose (Dextrose), HEPES, Phenol Red and Sodium Pyruvate.

5. The topical preparation for use according to any one of claims 1 to 4, wherein the microparticle is a PLGA microsphere and comprises:
a) a polymer ratio of PLGA 50:50;
b) a polymer ratio of PLGA 75:25; or
c) a polymer ratio of PLGA 85:15.

6. The topical preparation for use according to any one of claims 1-5, wherein the carrier-medium adjusts viscosity of the topical preparation to a range of 15 to 150 milli-Pascals per second.

7. The topical preparation for use according to claim 5, wherein the effective amount of diaminophenothiazine compound comprises 3,7-bis(dimethylamino) phenothiazin-5-ium chloride pre-loaded in the microparticle.

8. The topical preparation for use according to claim 5 or 7, wherein the microparticle is coated with a mucoadhesive agent such as chitosan.

9. The topical preparation for use according to any one of claims 1 to 8, wherein the peripheral nerve damage on a nerve site on the surface of the cornea comprises neurotrophic keratopathy.

10. The topical preparation for use according to any one of claims 1 to 8, wherein the use is for ophthalmic use and the body surface is a nerve site on the surface of the cornea.

11. The topical preparation for use according to any one of claims 1 to 8, wherein the peripheral nerves on body surfaces are on mucous membranes.

12. A topical preparation for preventing and/or treating peripheral nerve damage on body surfaces including mucous membranes or a nerve site on the surface of the cornea comprising, less than or equal to 150 µM of 3,7-bis(dimethylamino) phenothiazin-5-ium chloride and a carrier-medium comprising (i) a microparticle with a sustained release profile of 3,7-bis(dimethylamino) phenothiazin-5-ium chloride the microparticle comprising poly (lactide-co-glycolide); or (ii) a polymer selected from hydrophilic polymers of high molecular weight, polyvinyl alcohol, poloxamers, hyaluronic acid, carbomers, cellulose derivatives (gellan gum, xanthan gum), 2-hydroxypropyl-beta-cyclodextrin, poly(2-hydroxyethylmethacrylate), ethylene glycol dimethylacrylate, acrylamide, N-vinylpryrrolidone and ethylacrylate or combinations thereof, and wherein the viscosity of the preparation ranges from 15 to 150 milli-Pascals per second.

13. The topical preparation according to claim 12, wherein the carrier-medium comprises a microparticle that is a PLGA microsphere comprising:
a) a polymer ratio of PLGA 50:50
b) a polymer ratio of PLGA 75:25 or
c) a polymer ratio of PLGA 85:15.

14. The topical preparation according to claim 12 or 13, further comprising one or more additional compounds.

15. A kit comprising a) a topical composition comprising of an effective amount of 3,7-bis(dimethylamino) phenothiazin-5-ium chloride a carrier-medium comprising a microparticle with a sustained release profile of the 3,7-bis(dimethylamino) phenothiazin-5-ium chloride the microparticle comprising poly (lactide-co-glycolide), and b) a container for holding the said composition for use in a method of preventing and/or treating peripheral nerve damage on body surfaces including mucous membranes or a nerve site on the surface of the cornea, optionally wherein the container is configured to hold an amount of the said topical composition supporting a treatment for at least 1, 7,14, 21 or 30 days.

## Patentansprüche

1. Topisches Präparat zur Verwendung bei der Vorbeugung und/oder Behandlung von peripheren Nervenschäden auf Körperoberflächen, einschließlich Schleimhäuten oder einer Nervenstelle auf der Hornhautoberfläche, umfassend eine wirksame Menge von 3,7-bis(dimethylamino)phenothiazin-5-ium-chlorid und ein Trägermedium, umfassend (i) ein Mikropartikel mit einem verzögerten Freisetzungsprofil von 3,7-bis(dimethylamino)phenothiazin-5-ium-chlorid, wobei das Mikropartikel Poly(lactid-co-glycolid) umfasst; oder (ii) ein Polymer ausgewählt aus hydrophilen Polymeren mit hohem Molekulargewicht, Polyvinylalkohol, Poloxameren, Hyaluronsäure, Carbomeren, Cellulosederivaten (Gellangummi, Xanthangummi), 2-Hydroxypropyl-beta-cyclodextrin, Poly(2-hydroxyethylmethacrylat), Ethylenglycoldimethylacrylat, Acrylamid, N-Vinylpyrrolidon und Ethylacrylat oder Kombinationen davon.

2. Topisches Präparat zur Verwendung nach Anspruch 1, wobei das topische Präparat zwischen 0,0001 µM und 60 µM 3,7- bis(dimethylamino)phenothiazin-5-ium-chlorid umfasst, oder wobei das topische Präparat 0,0005 µM, 0,005 µM, 0,05 µM, 0,5 µM, 5 µM, 50 µM, oder 100 µM 3,7-bis(dimethylamino)phenothiazin-5-ium-chlorid umfasst.

3. Topisches Präparat zur Verwendung nach Anspruch 1, wobei das topische Präparat weniger als oder gleich 150 µM 3,7-bis(dimethylamino)phenothiazin-5-ium-chlorid umfasst.

4. Topisches Präparat zur Verwendung nach einem der Ansprüche 1 bis 3, weiter umfassend einen physiologischen Puffer, wobei der physiologische Puffer umfasst
a) Natriumbicarbonat und Natriumchlorid;
b) 4-(2-Hydroxyethyl)-1-piperazinethansulfonsäure; oder
c) ein neurobasales Medium ausgewählt aus der Gruppe bestehend aus Glycin, L-Alanin, L-Argininhydrochlorid, L-Asparagin-H₂O, L-Cystein, L-Histidinhydrochlorid-H₂O, L-Isoleucin, L-Leucin, L-Lysinhydrochlorid, L-Methionin, L-Phenylalanin, L-Prolin, L-Serin, L-Threonin, L-Tryptophan, L-Tyrosin, L-Valin, Cholinchlorid, D-Calciumpantothenat, Folsäure, Niacinamid, Pyridoxalhydrochlorid, Riboflavin, Thiaminhydrochlorid, Vitamin B12, i-Inositol, Calciumchlorid (CaCl₂) (wasserfrei), Eisennitrat (Fe(NO₃)*9H₂O), Magnesiumchlorid (wasserfrei), Kaliumchlorid (KCl), Natriumbicarbonat (NaHCO₃), Natriumchlorid (NaCl), Natriumphosphat monobasisch (NaH₂PO₄-H₂O), Zinksulfat (ZnSO₄*7H₂O), D-Glukose (Dextrose), HEPES, Phenolrot und Natriumpyruvat.

5. Topisches Präparat zur Verwendung nach einem der Ansprüche 1 bis 4, wobei das Mikropartikel eine PLGA-Mikrosphäre ist und umfasst:
a) ein Polymerverhältnis von PLGA 50:50;
b) ein Polymerverhältnis von PLGA 75:25; oder
c) ein Polymerverhältnis von PLGA 85:15.

6. Topisches Präparat zur Verwendung nach einem der Ansprüche 1 bis 5, wobei das Trägermedium die Viskosität des topischen Präparats auf einen Bereich von 15 bis 150 Milli-Pascal pro Sekunde einstellt.

7. Topisches Präparat zur Verwendung nach Anspruch 5, wobei die wirksame Menge der Diaminophenothiazin-Verbindung 3,7-bis(dimethylamino)phenothiazin-5-ium-chlorid umfasst, die in dem Mikropartikel vorgeladen ist.

8. Topisches Präparat zur Verwendung nach Anspruch 5 oder 7, wobei das Mikropartikel mit einem mukoadhäsiven Mittel wie Chitosan beschichtet ist.

9. Topisches Präparat zur Verwendung nach einem der Ansprüche 1 bis 8, wobei der periphere Nervenschaden an einer Nervenstelle auf der Hornhautoberfläche neurotrophische Keratopathie umfasst.

10. Topisches Präparat zur Verwendung nach einem der Ansprüche 1 bis 8, wobei die Verwendung für den ophthalmologischen Gebrauch ist und die Körperoberfläche eine Nervenstelle auf der Hornhautoberfläche ist.

11. Topisches Präparat zur Verwendung nach einem der Ansprüche 1 bis 8, wobei die peripheren Nerven auf Körperoberflächen auf Schleimhäuten sind.

12. Topisches Präparat zur Vorbeugung und/oder Behandlung von peripheren Nervenschäden auf Körperoberflächen, einschließlich Schleimhäuten oder einer Nervenstelle auf der Hornhautoberfläche, umfassend weniger als oder gleich 150 µM 3,7-bis(dimethylamino)phenothiazin-5-ium-chlorid und ein Trägermedium, umfassend (i) ein Mikropartikel mit einem verzögerten Freisetzungsprofil von 3,7-bis(dimethylamino)phenothiazin-5-ium-chlorid, wobei das Mikropartikel Poly(lactid-co-glycolid) umfasst; oder (ii) ein Polymer ausgewählt aus hydrophilen Polymeren mit hohem Molekulargewicht, Polyvinylalkohol, Poloxameren, Hyaluronsäure, Carbomeren, Cellulosederivaten (Gellangummi, Xanthangummi), 2-Hydroxypropyl-beta-cyclodextrin, Poly(2-hydroxyethylmethacrylat), Ethylenglycoldimethylacrylat, Acrylamid, N-Vinylpyrrolidon und Ethylacrylat oder Kombinationen davon, und wobei die Viskosität des Präparats im Bereich von 15 bis 150 Milli-Pascal pro Sekunde liegt.

13. Topisches Präparat nach Anspruch 12, wobei das Trägermedium ein Mikropartikel umfasst, das eine PLGA-Mikrosphäre ist umfassend:
a) ein Polymerverhältnis von PLGA 50:50
b) ein Polymerverhältnis von PLGA 75:25 oder
c) ein Polymerverhältnis von PLGA 85:15.

14. Topisches Präparat nach Anspruch 12 oder 13, weiter umfassend eine oder mehrere zusätzliche Verbindungen.

15. Kit, umfassend a) ein topisches Präparat, das eine wirksame Menge von 3,7-bis(dimethylamino)phenothiazin-5-ium-chlorid und ein Trägermedium umfasst, das ein Mikropartikel mit einem verzögerten Freisetzungsprofil des 3,7-bis(dimethylamino)phenothiazin-5-ium-chlorids umfasst, wobei das Mikropartikel Poly(lactid-co-glycolid) umfasst, und b) einen Behälter zum Aufbewahren des genannten Präparats zur Verwendung in einem Verfahren zur Vorbeugung und/oder Behandlung von peripheren Nervenschäden auf Körperoberflächen, einschließlich Schleimhäuten oder einer Nervenstelle auf der Hornhautoberfläche, wobei der Behälter optional so konfiguriert ist, dass er eine Menge des topischen Präparats enthält, die eine Behandlung für mindestens 1, 7, 14, 21 oder 30 Tage unterstützt.

## Revendications

1. Préparation topique pour une utilisation dans la prévention et/ou le traitement d'une lésion des nerfs périphériques sur les surfaces corporelles, y compris les membranes muqueuses ou un site nerveux sur la surface de la cornée, comprenant une quantité efficace de chlorure de 3,7-bis(diméthylamino)phénothiazin-5-ium et un milieu excipient comprenant (i) une microparticule ayant un profil de libération prolongée du chlorure de 3,7-bis(diméthylamino)phénothiazin-5-ium, la microparticule comprenant un poly(lactide-co-glycolide) ; ou (ii) un polymère choisi parmi les polymères hydrophiles à grande masse moléculaire, le poly(alcool vinylique), les poloxamères, l'acide hyaluronique, les carbomères, les dérivés de la cellulose (gomme gellane, gomme xanthane), la 2-hydroxypropyl-bêta-cyclodextrine, le poly(méthacrylate de 2-hydroxyéthyle), le diméthylacrylate d'éthylèneglycol, l'acrylamide, la N-vinylpryrrolidone et l'acrylate d'éthyle ou des combinaisons de ceux-ci.

2. Préparation topique pour une utilisation selon la revendication 1, la préparation topique comprenant entre 0,0001 µM et 60 µM de chlorure de 3,7-bis(diméthylamino)phénothiazin-5-ium, ou la préparation topique comprenant 0,0005 µM, 0,005 µM, 0,05 µM, 0,5 µM, 5 µM, 50 µM ou 100 µM de chlorure de 3,7-bis(diméthylamino)phénothiazin-5-ium.

3. Préparation topique pour une utilisation selon la revendication 1, la préparation topique comprenant une quantité ou égale à 150 µM de chlorure de 3,7-bis(diméthylamino)phénothiazin-5-ium.

4. Préparation topique pour une utilisation selon l'une des revendications 1 à 3, comprenant en outre un tampon physiologique, dans laquelle le tampon physiologique comprend
a) du bicarbonate de sodium et du chlorure de sodium ;
b) de l'acide 4-(2-hydroxyéthyl)-1-pipérazineéthanesulfonique ; ou
c) un milieu neurobasal choisi dans le groupe consistant en la glycine, la L-alanine, le chlorhydrate de L-arginine, la L-asparagine-H₂O, la L-cystéine, le chlorhydrate de L-histidine-H₂O, la L-isoleucine, la L-leucine, le chlorhydrate de L-lysine, la L-méthionine, la L-phénylalanine, la L-proline, la L-sérine, la L-thréonine, le L-tryptophane, la L-tyrosine, la L-valine, le chlorure de choline, le D-pantothénate de calcium, l'acide folique, le niacinamide, le chlorhydrate de pyridoxal, la riboflavine, le chlorhydrate de thiamine, la vitamine B12, l'i-inositol, le chlorure de calcium (CaCl₂) (anhydre), le nitrate ferrique (Fe (NO₃)*9H₂O), le chlorure de magnésium (anhydre), le chlorure de potassium (KCl), le bicarbonate de sodium (NaHCO₃), le chlorure de sodium (NaCl), le phosphate de sodium monobasique (NaH₂PO₄-H₂O), le sulfate de zinc (ZnSO₄*7H₂O), le D-glucose (dextrose), le HEPES, le rouge phénol et le pyruvate de sodium.

5. Préparation topique pour une utilisation selon l'une des revendications 1 à 4, dans laquelle la microparticule est une microsphère de PLGA et comprend :
a) un rapport entre monomères du PLGA de 50:50 ;
b) un rapport entre monomères du PLGA de 75:25 ; ou
c) un rapport entre monomères du PLGA de 85:15.

6. Préparation topique pour une utilisation selon l'une des revendications 1 à 5, dans laquelle le milieu excipient ajuste la viscosité de la préparation topique dans une plage de 15 à 150 millipascals par seconde.

7. Préparation topique pour une utilisation selon la revendication 5, dans laquelle la quantité efficace du composé diaminophénothiazine comprend du chlorure de 3,7-bis(diméthylamino)phénothiazin-5-ium préchargé dans la microparticule.

8. Préparation topique pour une utilisation selon la revendication 5 ou 7, dans laquelle la microparticule est revêtue d'un agent muco-adhésif tel que le chitosane.

9. Préparation topique pour une utilisation selon l'une des revendications 1 à 8, la lésion des nerfs périphériques sur un site nerveux sur la surface de la cornée comprenant une kératopathie neurotrophique.

10. Préparation topique pour une utilisation selon l'une des revendications 1 à 8, l'utilisation portant sur une utilisation ophtalmique, et la surface corporelle étant un site nerveux sur la surface de la cornée.

11. Préparation topique pour une utilisation selon l'une des revendications 1 à 8, les nerfs périphériques sur les surfaces corporelles se trouvant sur des membranes muqueuses.

12. Préparation topique pour prévenir et/ou traiter une lésion des nerfs périphériques sur les surfaces corporelles comprenant les membranes muqueuses ou un site nerveux sur la surface de la cornée, comprenant une quantité inférieure ou égale à 150 µM de chlorure de 3,7-bis(diméthylamino)phénothiazin-5-ium et un milieu excipient comprenant (i) une microparticule ayant un profil de libération prolongée du chlorure de 3,7-bis(diméthylamino)phénothiazin-5-ium, la microparticule comprenant un poly(lactide-co-glycolide) ; ou (ii) un polymère choisi parmi les polymères hydrophiles à grande masse moléculaire, le poly(alcool vinylique), les poloxamères, l'acide hyaluronique, les carbomères, les dérivés de la cellulose (gomme gellane, gomme xanthane), la 2-hydroxypropyl-bêta-cyclodextrine, le poly(méthacrylate de 2-hydroxyéthyle), le diméthylacrylate d'éthylèneglycol, l'acrylamide, la N-vinylpryrrolidone et l'acrylate d'éthyle ou des combinaisons de ceux-ci,
et la viscosité de la préparation étant comprise dans la plage de 15 à 150 millipascals par seconde.

13. Préparation topique selon la revendication 12, dans laquelle le milieu excipient comprend une microparticule qui est une microsphère de PLGA comprenant :
a) un rapport entre monomères du PLGA de 50:50,
b) un rapport entre monomères du PLGA de 75:25, ou
c) un rapport entre monomères du PLGA de 85:15.

14. Préparation topique selon la revendication 12 ou 13, comprenant en outre un ou plusieurs composés additionnels.

15. Nécessaire comprenant a) une composition topique comprenant une quantité efficace de chlorure de 3,7-bis(diméthylamino)phénothiazin-5-ium, un milieu excipient comprenant une microparticule ayant un profil de libération prolongée du chlorure de 3,7-bis(diméthylamino)phénothiazin-5-ium, la microparticule comprenant un poly(lactide-co-glycolide), et b) un récipient destiné à contenir ladite composition pour une utilisation dans une méthode de prévention et/ou de traitement d'une lésion des nerfs périphériques sur des surfaces corporelles comprenant les membranes muqueuses ou un site nerveux sur la surface de la cornée, éventuellement dans laquelle le récipient est conçu pour contenir une quantité de ladite composition topique convenant à un traitement pendant au moins 1, 7, 14, 21 ou 30 jours.
